# EUROPEAN PATENT SPECIFICATION

(11) **EP 3 370 649 B1**
(45) Date of publication and mention of the grant of the patent: **15.03.2023**
(21) Application number: 16862838.6
(22) Date of filing: 02.11.2016
(51) Int. Cl.: A61F 2/24

(54) **DEVICES FOR REDUCING CARDIAC VALVE REGURGITATION**
VORRICHTUNGEN ZUR REDUZIERUNG VON HERZKLAPPENREGURGITATION
DISPOSITIFS DE RÉDUCTION DE RÉGURGITATION VALVULAIRE CARDIAQUE

(30) Priority: 02.11.2015 US 201562249815 P; 30.12.2015 US 201562273313 P; 04.02.2016 US 201662291406 P
(43) Date of publication of application: 12.09.2018
(73) Proprietor: Edwards Lifesciences Corporation, Irvine, CA 92614 (US); Tricardia, LLC, Excelsior, Minnesota 55331 (US); Schwartz, Robert S., Inver Grove Heights, Minnesota 55076 (US); Van Tassel, Robert A., Excelsior, Minnesota 55331 (US); Rowe, Stanton J., Newport Coast, California 92657-1812 (US)
(72) Inventor: SCHWARTZ, Robert S., Inver Grove Heights, Minnesota 55076 (US); VAN TASSEL, Robert A., Excelsior, Minnesota 55331 (US); ROWE, Stanton J., Newport Coast, California 92657-1812 (US)
(74) Representative: Eisenführ Speiser
(86) International application number: PCT/US2016/060035
(87) International publication number: WO 2017/079234

(56) References cited:
- US-A1- 2005 149 178
- US-A1- 2007 185 571
- US-A1- 2010 145 440
- US-A1- 2010 262 233
- US-A1- 2011 257 728
- US-A1- 2013 310 928
- US-A1- 2013 325 110
- US-A1- 2013 325 110
- US-A1- 2014 031 928

## Description

### FIELD OF THE INVENTION

The present invention relates generally to devices for improving the function of a defective heart valve. The devices disclosed herein are particularly well adapted for implantation in a patient's heart for reducing regurgitation through a heart valve.

### BACKGROUND OF THE INVENTION

The function of the heart may be seriously impaired if any of the heart valves are not functioning properly. The heart valves may lose their ability to close properly due to e.g. dilation of an annulus around the valve, ventricular dilation, or a leaflet being flaccid causing a prolapsing leaflet. The leaflets may also have shrunk due to disease, e.g. rheumatic disease, and thereby leave a gap in the valve between the leaflets. The inability of the heart valve to close properly can cause a leak backwards (i.e., from the outflow to the inflow side), commonly referred to as regurgitation, through the valve. Heart valve regurgitation may seriously impair the function of the heart since more blood will have to be pumped through the regurgitating valve to maintain adequate circulation. Heart valve regurgitation decreases the efficiency of the heart, reduces blood circulation, and adds stress to the heart. In early stages, heart valve regurgitation leaves a person fatigued or short of breath. If left unchecked, the problem can lead to congestive heart failure, arrhythmias or death.

Heart valve disease, such as valve regurgitation, is typically treated by replacing or repairing the diseased valve during open-heart surgery. However, open-heart surgery is highly invasive and is therefore not an option for many patients. For high-risk patients, a less-invasive method for repair of heart valves is considered generally advantageous.

Devices for reducing regurgitation through a heart valve are known from US 2013/0325110 A1, US 2007/0185571 A1, and US 2014/0031928 A1. Annuloplasty devices are known from US 2011/0257728 A1, US 2005/0149178 A1, and US 2010/0145440 A1.

In particular, US 2005/0149178 A1 discloses a device for repairing a heart valve which comprises an implantation instrument. The implantation instrument comprises a first support ring, and a second support ring connected to said first support ring to form a coiled configuration. The first support ring is configured to abut one side of the valve and the second support ring is configured to abut an opposite side of the valve to thereby trap a portion of the valve tissue therebetween. The device further comprises an annuloplasty implant adapted to be attached to the heart valve annulus in order to reshape the annulus and allow the leaflets to open and close properly. The annuloplasty implant is connected to the implantation instrument for insertion to the annulus.

Moreover, US patent application 2010/0145440 A1 discloses a device for improving the function of a heart valve which comprises a first loop-shaped support, which is configured to abut a first side of the heart valve, and a first flange unit being connected to said first loop-shaped support. The flange unit is configured to be arranged against said annulus when said first loop-shaped support is abutting said heart valve.

### SUMMARY

The present invention is defined in the appended claims. Heart valve regurgitation can be reduced by sizing a coapting element to provide a gap between the coapting element and a heart valve when the heart is in a diastolic phase. The size of the coapting element can also be selected such that the heart valve seals against the coapting element when the heart is in the systolic phase. The coapting element can allow flow through the coapting element when the heart is in a diastolic phase and prevents flow through the coapting element when the heart is in a systolic phase.

The coapting element is part of a valved regurgitation reduction device that includes the coapting element and a valve coupled to the coapting element. The valve coupled to the coapting element is configured to open and allow flow through the coapting element when the heart is in a diastolic phase and to close and prevent flow through the coapting element when the heart is in the systolic phase.

A further understanding of the nature and advantages of the present invention are set forth in the following description and claims, particularly when considered in conjunction with the accompanying drawings in which like parts bear like reference numerals.

### BRIEF DESCRIPTION OF THE DRAWINGS

To further clarify various aspects of the present disclosure, a more particular description of the certain embodiments and examples will be made by reference to various aspects of the appended drawings. It is appreciated that these drawings depict only typical embodiments and examples of the present disclosure and are therefore not to be considered limiting of the scope of the disclosure. Moreover, while the figures may be drawn to scale for some cases, the figures are not necessarily drawn to scale for all cases. The present disclosure will be described and explained with additional specificity and detail through the use of the accompanying drawings.
Figure 1A is a cutaway view of the human heart in a diastolic phase schematically showing a valved regurgitation reduction device positioned in the tricuspid valve for reducing tricuspid valve regurgitation;
Figure 1B is a sectional view taken along the plane indicated by lines 1B-1B in Figure 1A;
Figure 2A is a cutaway view of the human heart and valved regurgitation reduction device of Figure 1A in a systolic phase;
Figure 2B is a sectional view taken along the plane indicated by lines 2B-2B in Figure 2A;
Figure 3 is a cutaway view of the human heart in a diastolic phase schematically showing a valved regurgitation reduction device positioned in the mitral valve for reducing mitral valve regurgitation;
Figure 4 is a cutaway view of the human heart and valved regurgitation reduction device of Figure 3 in a systolic phase;
Figures 5-11 illustrate examples of valve types that may be included in the valved regurgitation reduction device;
Figure 12A is a cutaway view of the human heart in a diastolic phase showing an expandable valved regurgitation reduction device positioned in the tricuspid valve for reducing tricuspid valve regurgitation;
Figure 12B is a sectional view taken along the plane indicated by lines 12B-12B in Figure 12A;
Figure 13A is a cutaway view of the human heart and expandable valved regurgitation reduction device of Figure 12A in a systolic phase;
Figure 13B is a sectional view taken along the plane indicated by lines 13B-13B in Figure 13A;
Figure 14A is a cutaway view of the human heart in a diastolic phase showing an expandable valved regurgitation reduction device positioned in the mitral valve for reducing mitral valve regurgitation;
Figure 14B is a cutaway view of the human heart and expandable valved regurgitation reduction device of Figure 14A in a systolic phase;
Figure 15A is a cutaway view of the human heart in a diastolic phase showing introduction of an anchoring catheter into the right ventricle;
Figure 15B is a cutaway view of the human heart in a systolic phase showing retraction of the anchoring catheter after installing a device anchor at the apex of the right ventricle;
Figure 16A illustrates the beginning of deployment of a valved coaptation device in a tricuspid valve;
Figure 16B is a sectional view of the right atrium and ventricle of a heart in a diastolic phase that illustrate a deployed valved regurgitation reduction device on an anchor rail to position the valved regurgitation reduction device within the tricuspid valve;
Figure 16C is a sectional view of the heart and valved regurgitation reduction device of Figure 16B where the heart is in a systolic phase;
Figure 16D is a sectional view of the right atrium and ventricle of a heart in a diastolic phase that illustrate a deployed valved regurgitation reduction device on another anchor rail to position the valved regurgitation reduction device within the tricuspid valve;
Figure 16E is a sectional view of the heart and valved regurgitation reduction device of Figure 16D where the heart is in a systolic phase;
Figures 17A-17C illustrate examples of strut frames for positioning and holding a valved regurgitation reduction device on an anchor rail;
Figure 18A illustrates the beginning of deployment of a valved coaptation device in a tricuspid valve;
Figure 18B is a sectional view of the right atrium and ventricle of a heart in a diastolic phase that illustrate a deployed valved regurgitation reduction device on an anchor rail to position the valved regurgitation reduction device within the tricuspid valve;
Figure 18C is a sectional view of the heart and valved regurgitation reduction device of Figure 18B where the heart is in a systolic phase;
Figure 18D is a sectional view of the right atrium and ventricle of a heart in a diastolic phase that illustrate a deployed valved regurgitation reduction device on another anchor rail to position the valved regurgitation reduction device within the tricuspid valve;
Figure 18E is a sectional view of the heart and valved regurgitation reduction device of Figure 18D where the heart is in a systolic phase;
Figure 19A is a view taken along the plane indicated by lines 19A-19A in Figure 18B when the heart is in a diastolic phase;
Figure 19B is a view taken along the plane indicated by lines 19B-19B in Figure 18C when the heart is in a systolic phase;
Figure 20 is a broader view of a valved regurgitation reduction device with the valved regurgitation reduction device positioned within the tricuspid valve and a proximal length of the delivery catheter including a locking collet shown exiting the subclavian vein to remain implanted subcutaneously;
Figure 21 is a sectional view of the heart that illustrates an expandable valved regurgitation reduction device mounted to positioning wires to position the expandable valved regurgitation reduction device within the tricuspid valve;
Figure 22A is a view taken along the plane indicated by lines 22-22 in Figure 21 when the heart is in a diastolic phase;
Figure 22B is a view taken along the plane indicated by lines 22-22 in Figure 21 when the heart is in a systolic phase;
Figure 23 is a sectional view of the right atrium and ventricle that illustrate an expandable valved regurgitation reduction device externally secured to an anchor to position the expandable valved regurgitation reduction device within the tricuspid valve;
Figure 24A illustrates the beginning of deployment of a valved regurgitation reduction device in a tricuspid valve;
Figure 24B is a sectional view of the right atrium and ventricle of a heart in a diastolic phase that illustrate a deployed valved regurgitation reduction device on an anchor rail to position the valved regurgitation reduction device within the tricuspid valve;
Figure 24C is a sectional view of the heart and valved regurgitation reduction device of Figure 24B where the heart is in a systolic phase;
Figure 25A is a view taken along the plane indicated by lines 25A-25A in Figure 24B illustrating the heart and the expandable valved regurgitation reduction device when the heart is in a diastolic phase;
Figure 25B is a view taken along the plane indicated by lines 25B-25B in Figure 24C illustrating the heart and the expandable valved regurgitation reduction device when the heart is in a systolic phase;
Figure 26 illustrates a catheter anchored to the apex of the right ventricle using an L-shaped stabilizing catheter secured within a coronary sinus;
Figure 27 schematically illustrates a stabilizing rod extending laterally from a valved regurgitation reduction device in the right atrium above the tricuspid valve;
Figure 28 illustrates an adjustable stabilizing rod mounted on a delivery catheter and secured within the coronary sinus;
Figure 29 illustrates an alternative delivery catheter having a pivot joint just above the valved regurgitation reduction device;
Figures 30 and 31 show two ways to anchor a delivery catheter to the superior vena cava for stabilizing the valved regurgitation reduction device;
Figures 32 and 33 show a valved regurgitation reduction device having pull wires extending through it for altering the position of the valved regurgitation reduction device within the tricuspid valve leaflets;
Figure 34 shows a valved regurgitation reduction device anchored with stents in both the superior and inferior vena cava and having rods connecting the stents to the atrial side of the valved regurgitation reduction device;
Figures 35-37 are schematic views of a valved regurgitation reduction device mounted for lateral movement on a flexible delivery catheter that collapses and allows rotation for seating the valved regurgitation reduction device centrally in the valve plane even if the delivery catheter is not centered in the valve.
Figure 38 is a cutaway view of the human heart in a diastolic phase schematically showing a device that reduces the size of a valve annulus, and a valved regurgitation reduction device positioned in the tricuspid valve for reducing tricuspid valve regurgitation;
Figure 39 illustrates the heart, a device that reduces the size of a valve annulus, and a valved regurgitation reduction device in a systolic phase; and
Figures 40-43 illustrate installation of a shape memory support ring in a valve annulus.

### DETAILED DESCRIPTION

The following description refers to the accompanying drawings, which illustrate specific embodiments and examples of the the present disclosure. Other embodiments having different structures and operation do not depart from the scope of the present invention.

The present disclosure is directed to devices for improving the function of a defective heart valve. It should be noted that various valved regurgitation reduction devices and systems for delivery and implant are disclosed herein, and any combination of these options may be made unless specifically excluded. For example, any of the valved regurgitation reduction devices disclosed, with any type of valve, may be combined with any of the flexible rail anchors, even if a specific combination is not explicitly described. Likewise, the different constructions of valved regurgitation reduction devices may be mixed and matched, such as by combining any valve type, tissue cover, etc. with any anchor, even if not explicitly disclosed. In short, individual components of the disclosed systems may be combined unless mutually exclusive or otherwise physically impossible.

For the sake of uniformity, in these figures and others in the application the valved regurgitation reduction devices are depicted such that the atrial end is up, while the ventricular end is down. These directions may also be referred to as "proximal" as a synonym for up or the atrial end, and "distal" as a synonym for down or the ventricular end, which are terms relative to the physician's perspective.

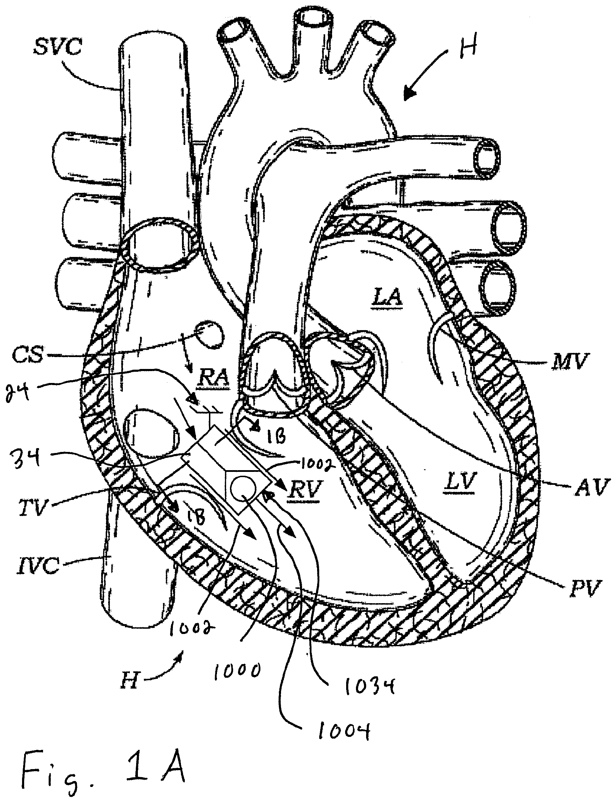

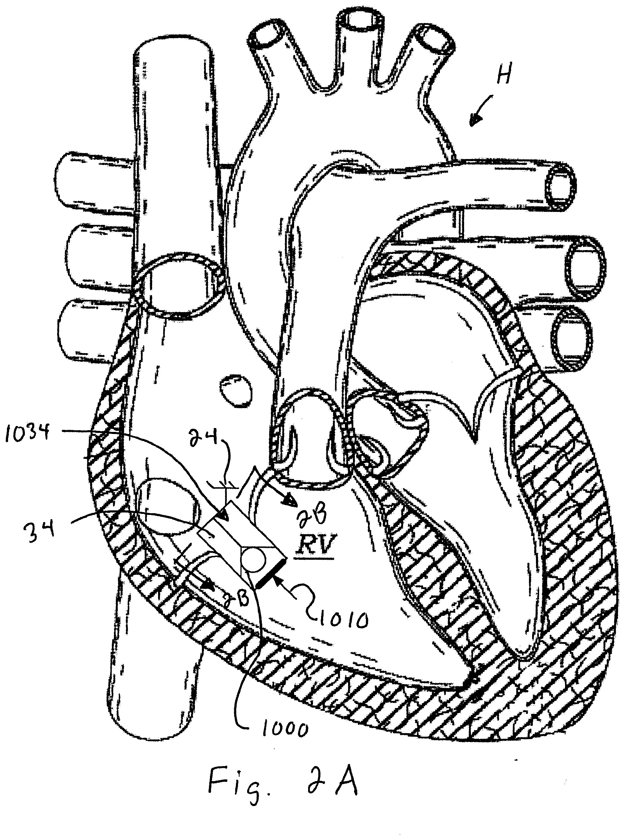

Figures 1A and 2A are cutaway views of the human heart H in diastolic and systolic phases, respectively. The right ventricle RV and left ventricle LV are separated from the right atrium RA and left atrium LA, respectively, by the tricuspid valve TV and mitral valve MV; i.e., the atrioventricular valves. Additionally, the aortic valve AV separates the left ventricle LV from the ascending aorta (not identified) and the pulmonary valve PV separates the right ventricle from the pulmonary artery (also not identified). Each of these valves has flexible leaflets extending inward across the respective orifices that come together or "coapt" in the flowstream to form the one-way, fluid-occluding surfaces. The regurgitation reduction devices of the present application are described primarily with respect to the atrioventricular valves, and in particular the tricuspid valve. Therefore, anatomical structures of the right atrium RA and right ventricle RV will be explained in greater detail, though it should be understood that the devices described herein may equally be used to treat the mitral valve MV.

The right atrium RA receives deoxygenated blood from the venous system through the superior vena cava SVC and the inferior vena cava IVC, the former entering the right atrium from above, and the latter from below. The coronary sinus CS is a collection of veins joined together to form a large vessel that collects deoxygenated blood from the heart muscle (myocardium), and delivers it to the right atrium RA. During the diastolic phase, or diastole, seen in Figure 1A, the venous blood that collects in the right atrium RA enters the tricuspid valve TV by expansion of the right ventricle RV. In the systolic phase, or systole, seen in Figure 2A, the right ventricle RV contracts to force the venous blood through the pulmonary valve PV and pulmonary artery into the lungs. During systole, the leaflets of the tricuspid valve TV close to prevent the venous blood from regurgitating back into the right atrium RA. It is during systole that regurgitation through the tricuspid valve TV becomes an issue, and then that the devices of the present application are most beneficial.

### Regurgitation Reduction System

Referring to Figures 1A and 2A, a regurgitation reduction system includes a valved regurgitation reduction device 1034 and a device anchor 24. In Figure 1A, the valved regurgitation reduction device 1034 is placed in the tricuspid valve TV and held in place in the tricuspid valve TV by the anchor 24. The valved regurgitation reduction device 1034 can take a wide variety of forms. The illustrated valved regurgitation reduction device 1034 includes a valve 1000 (schematically illustrated check valve that can have any physical configuration) coupled to a coapting element 34.

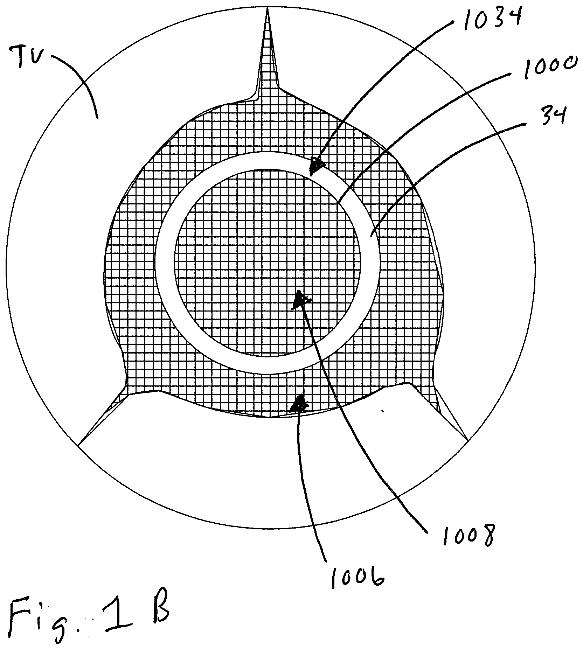

Referring to Figures 1A and 1B, when the heart is in the diastolic phase, the valve 1000 opens and the tricuspid valve TV opens around the coapting element 34 of the valved regurgitation reduction device 1034. Blood flows from the right atrium RA to the right ventricle RV between the tricuspid valve TV and the coapting element 34 as indicated by arrows 1002 and/or through the valve 1000 as indicated by arrow 1004. Figure 1B illustrates space 1006 between the coapting element 34 and the tricuspid valve TV. The blank space 1008 in the coapting element 34 represents the valve 1000 being open when the heart is in the diastolic phase. The cross-hatching in Figure 1B illustrates areas through which blood flows. Cross-hatching similar to that shown in Figure 1B represents blood flow in other figures, unless otherwise indicated.

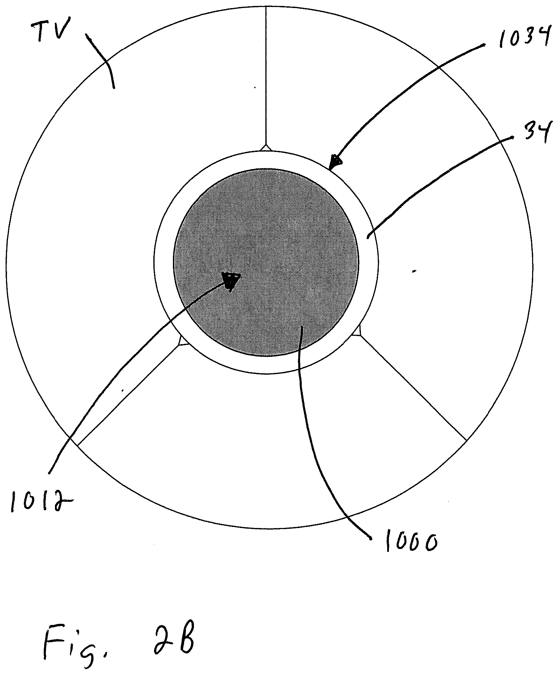

Referring to Figures 2A and 2B, when the heart is in the systolic phase, the valve 1000 closes and the tricuspid valve TV closes around the coapting element 34 of the valved regurgitation reduction device 1034. Blood flow from the right ventricle RV to the right atrium RA is blocked by the tricuspid valve TV closing on the coapting element 34 and by the valve 1000 being closed and blocking blood flow as indicated by arrow 1010. Figure 2B illustrates the tricuspid valve sealing against the coapting element 34 and the tricuspid valve TV. The solid area 1012 in the coapting element 34 represents the valve 1000 being closed when the heart is in the systolic phase.

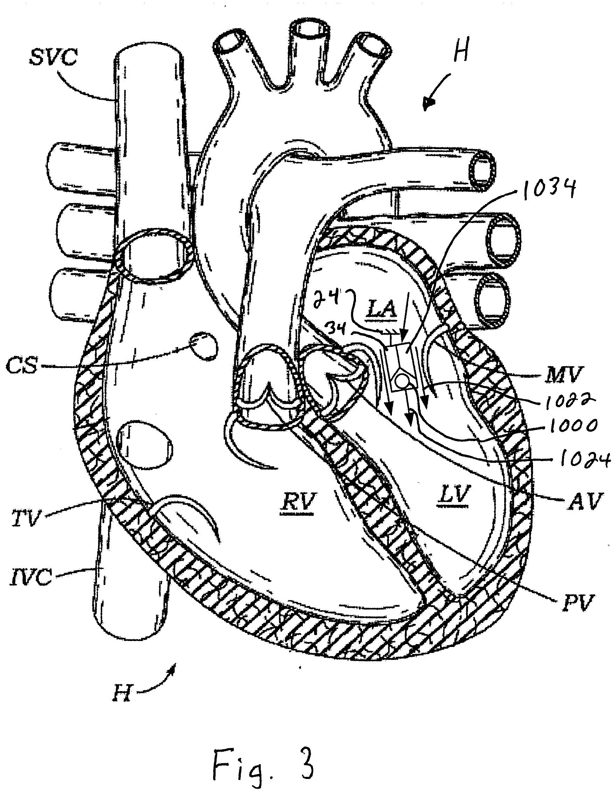

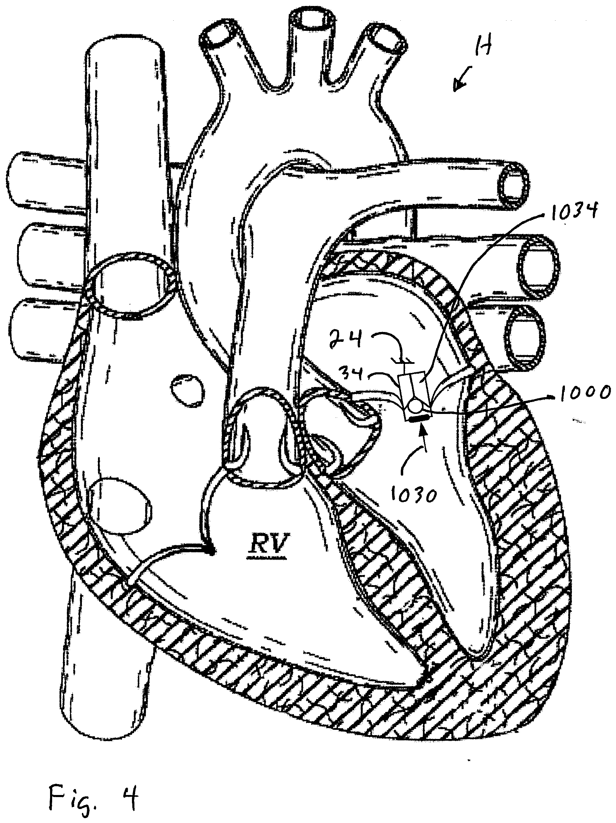

The valved regurgitation reduction device 1034 can be adapted to reduce regurgitation of any heart valve. For example, in Figures 3 and 4 the valved regurgitation reduction device 1034 is placed in the mitral valve MV and held in place in the mitral valve MV by the anchor 24. Referring to Figure 3, when the heart is in the diastolic phase, the valve 1000 opens and the mitral valve MV opens around the coapting element 34 of the valved regurgitation reduction device 1034. Blood flows from the left atrium LA to the left ventricle LV between the mitral valve MV and the coapting element 34 as indicated by arrows 1022 and through the valve 1000 as indicated by arrow 1024.

Referring to Figure 4, when the heart is in the systolic phase, the valve 1000 closes and the mitral valve MV closes around the coapting element 34 of the valved regurgitation reduction device 1034. Blood flow from the left ventricle LV to the left atrium LA is blocked by the mitral valve MV closing on the coapting element 34 and by the valve 1000 being closed and blocking blood flow as indicated by arrow 1030.

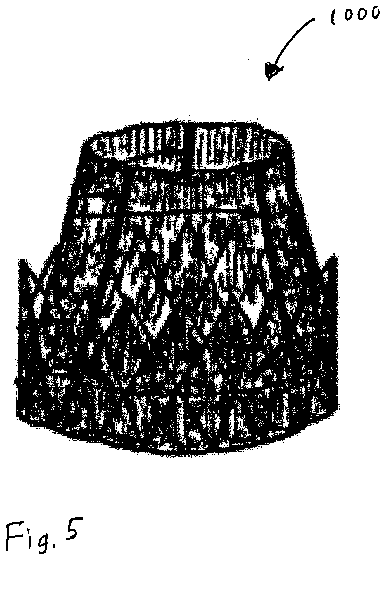

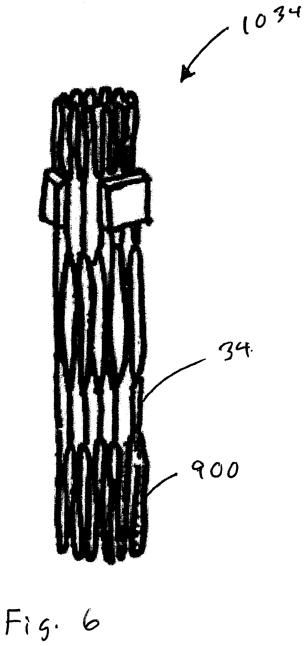

The valve 1000 of the valved regurgitation reduction device 1034 can take a wide variety of different forms. The valve 1000 can be configured to be installed transvascularly in the heart along with the coapting element 34. For example, the valve 1000 and coapting element 34 may be expandable and collapsible to facilitate transvascular application in a heart. Alternatively, however, the valve 1000 may be configured for surgical application. Figures 5-11 illustrate a few of the many valve configurations that may be used. Any valve type may be used and some valves that are traditionally applied surgically may be modified for transvascular installation. Figure 5 illustrates an expandable valve for transvascular installation that is shown and described in US Patent No. 8,002,825. An example of a tri-leaflet valve is shown and described in Published Patent Cooperation Treaty Application No. WO 2000/42950. An example of a tri-leaflet valve is shown and described in US Patent No. 5,928,281. An example of a tri-leaflet valve is shown and described in US Patent No. 6,558,418. Figures 6-8 illustrate an example of an expandable tri-leaflet valve. An example of an expandable tri-leaflet valve is the Edwards SAPIEN Transcatheter Heart Valve.

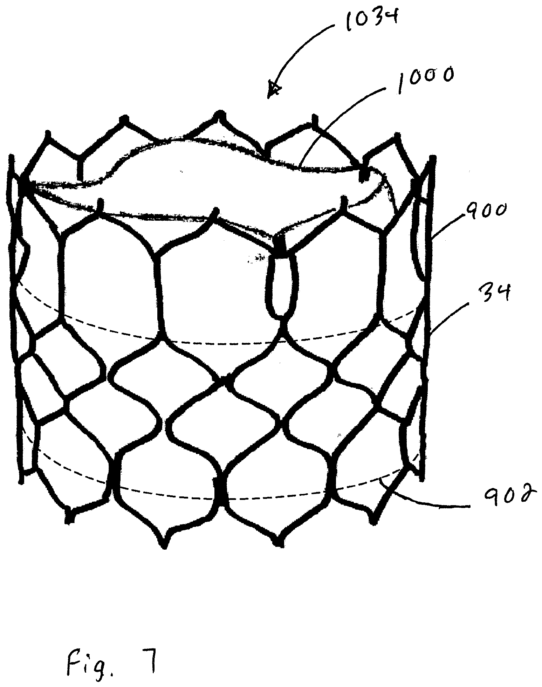

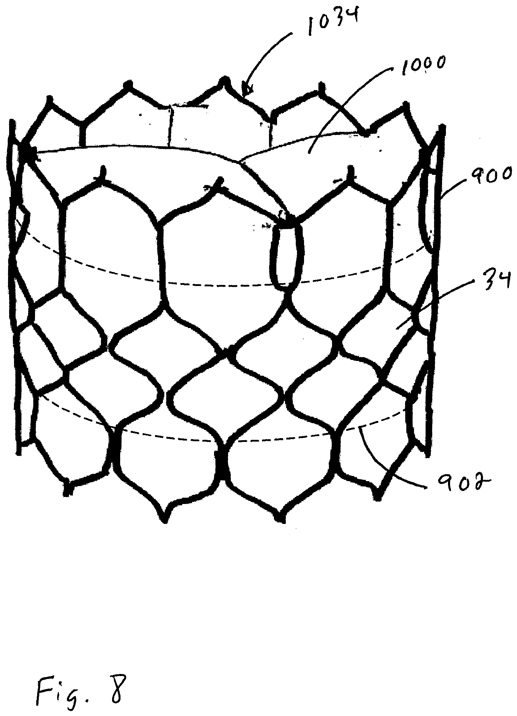

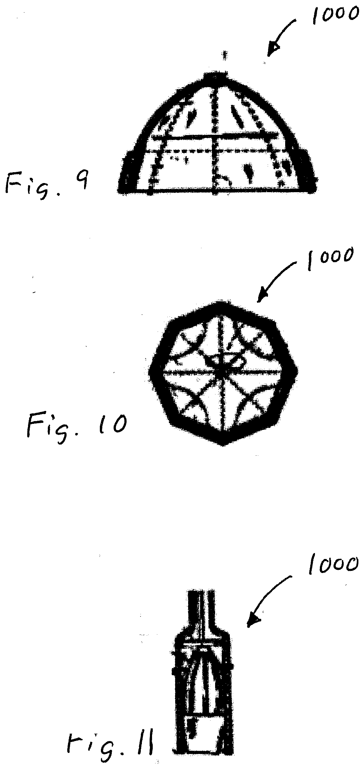

The coapting element 34 may comprise a cage 900 of the expandable tri-leaflet valve with a covering 902 (see Figures 7 and 8). The covering 902 may cover the entire cage or a portion of it. For example, the covering 902 may be configured to cover the portion of the cage 900 that is engaged by the tricuspid or mitral valve. In the example illustrated by Figure 6, the valve is a tri-leaflet valve compressed inside the cage 900. Figure 7 illustrates the cage 900 expanded and the valve 1000 in an open condition. Figure 8 illustrates the cage 900 expanded and the valve 1000 in a closed condition. Figures 9-11 illustrate an example of an expandable valve that is shown and described in US Patent No. 6,540,782. An example of a valve is shown and described in US Patent No. 3,365,728. An example of a valve is shown and described in US Patent No. 3,824,629. An example of a valve is shown and described in US Patent No. 5,814,099. Note that the covering 902 in particular is optional, and may or not be present at all.

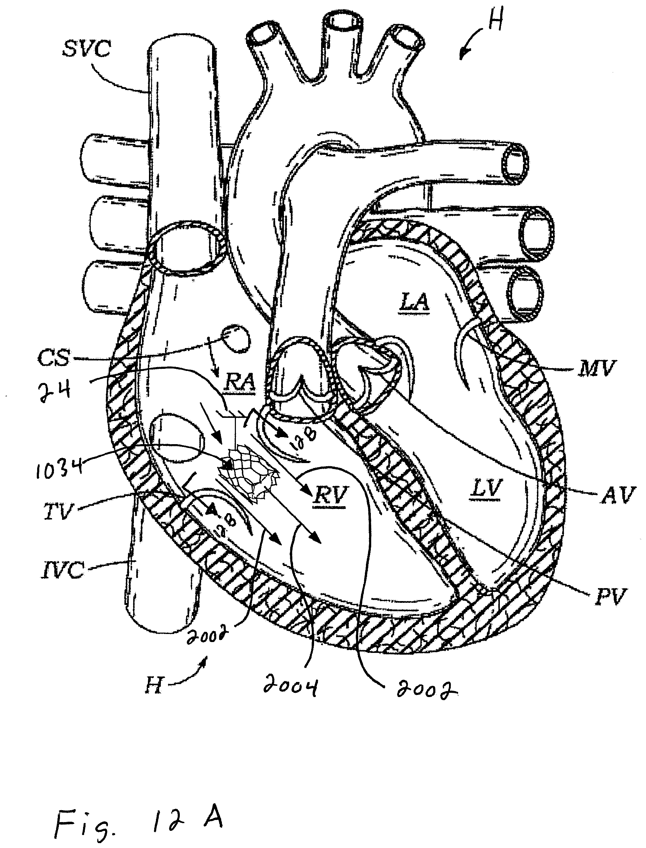

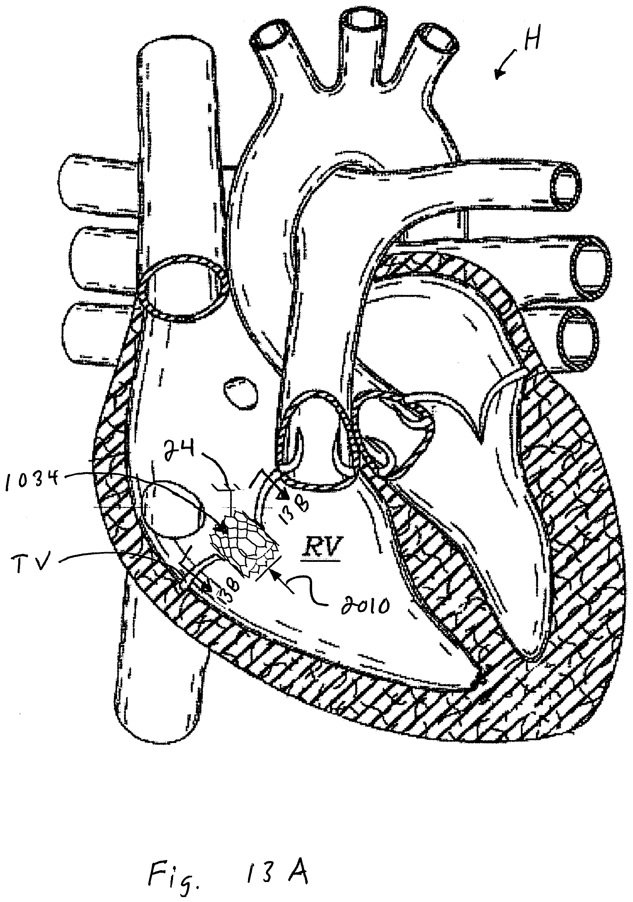

Referring to Figures 12A and 13A, a regurgitation reduction system can include a valved regurgitation reduction device 1034 and a device anchor 24. In the example illustrated by Figure 12A, the valved regurgitation reduction device 1034 is placed in the tricuspid valve TV and held in place by the anchor 24. In the example illustrated by Figures 12A and 13A, the valved regurgitation reduction device 1034 includes the valve 1000 of Figures 6-8 and the coapting element 34 comprises the cage 900, and cover 902 illustrated by Figures 6-8. The cage 900 can take a wide variety of different forms. Figures 5-8, 9-11, 17B, and 17C illustrate a few of the possible cage configurations. However, any cage configuration capable of coapting with a native heart valve and supporting an artificial valve 1000 can be used.

The cover 902 can take a wide variety of forms. The cover 902 can comprise one or more panels of bioprosthetic tissue sewn around portions of the frame 900. The cover 902 may be formed of a variety of xenograft sheet tissue, though bovine pericardial tissue is particularly preferred for its long history of use in cardiac implants, physical properties and relative availability. Other options are porcine or equine pericardium, for example. The cover 902 can be bioprosthetic tissue, such as bovine pericardium with a smooth side of the pericardium placed facing outward and a rough side facing inward. In the example illustrated by Figures 6-8, the cover 902 has a proximal end that is open to fluid flow and a distal end that is also open. This allows blood to flow through the coapting element 34 when the valve 1000 is open. During diastole, blood flows around the coapting element 34. Then during systole, as the native leaflets close and contact the coapting element and the pressure and blood flow work to fill the interior of the coapting element by pushing blood in, the interior of the coapting element is at the same pressure as that in the RV and a seal is created. These phases of the cardiac cycle are common to both the tricuspid and mitral valves.

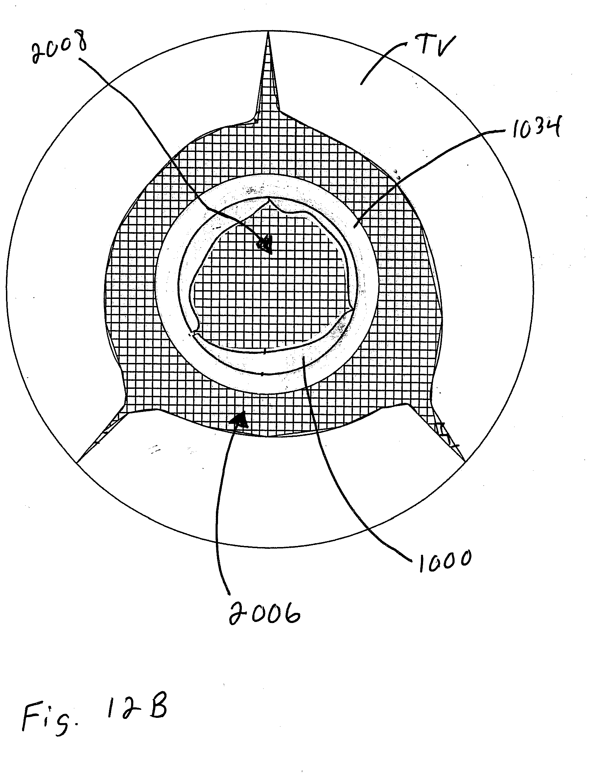

Referring to Figures 12A and 12B, when the heart is in the diastolic phase, the valve 1000 opens and the tricuspid valve TV opens around the cage 900 and cover 902 of the valved regurgitation reduction device 1034. Blood flows from the right atrium RA to the right ventricle RV between the tricuspid valve TV and the cage 900 and cover 902 as indicated by arrows 2002 and/or through the valve 1000 as indicated by arrow 2004. Figure 12B illustrates space 2006 between the cage 900 and cover 902 and the tricuspid valve TV. The blank space 2008 represents the valve 1000 being open when the heart is in the diastolic phase. As before, the cross-hatching in Figure 12B illustrates areas of blood flow.

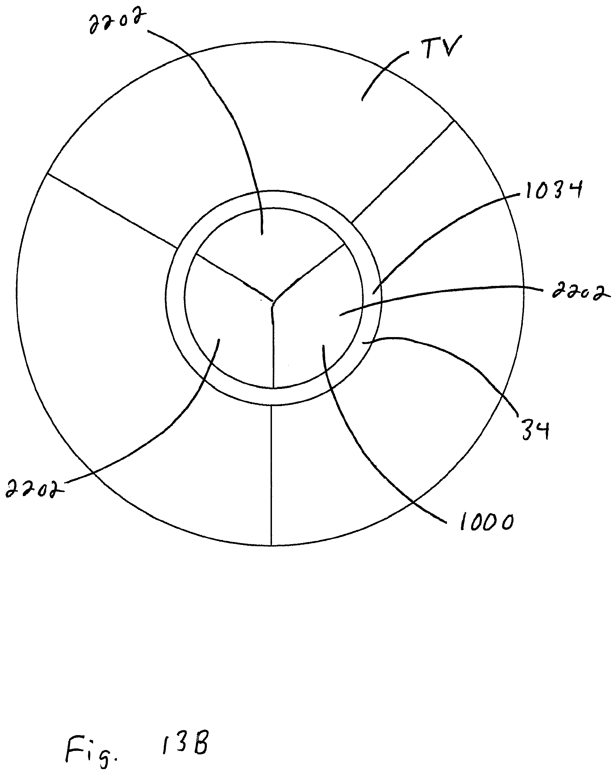

Referring to Figures 13A and 13B, when the heart is in the systolic phase, the valve 1000 closes and the tricuspid valve TV closes around the cage 900 and cover 902 of the valved regurgitation reduction device 1034. Blood flow from the right ventricle RV to the right atrium RA is blocked by the tricuspid valve TV closing on the cage 900 and cover 902, and by the valve 1000 being closed and blocking blood flow as indicated by arrow 2010. Figure 13B illustrates the tricuspid valve sealing against the cage 900 and cover 902. The tricuspid valve TV and the illustrated three cusps 2202 of the valve 1000 are shown as closed when the heart is in the systolic phase. A covering of pericardium or polymeric material may be provided over the cage to prevent abrasion of the leaflets against the cage. This covering can also serve to create a larger surface during coaptation.

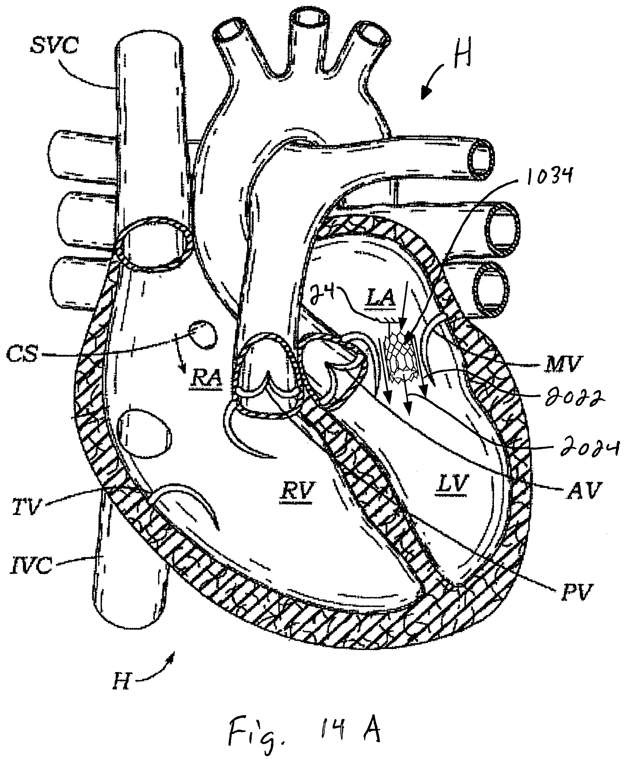

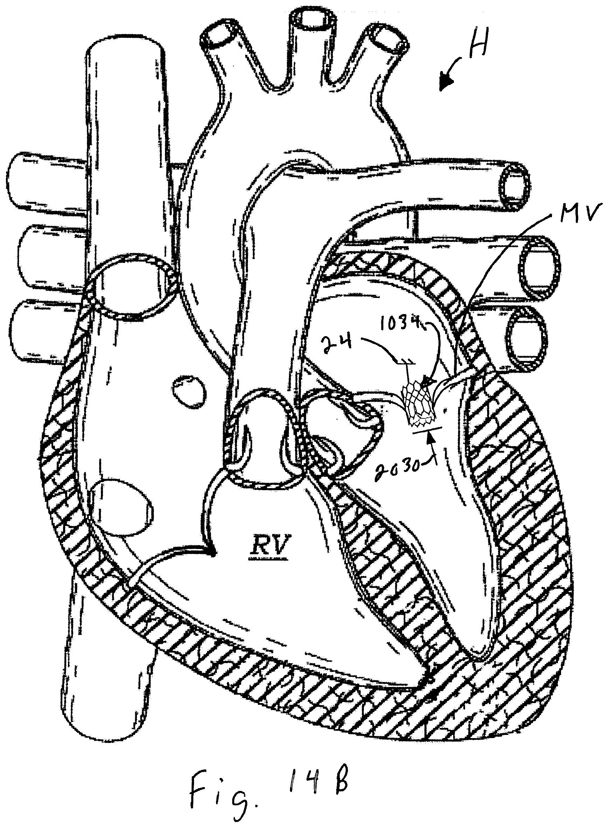

The valved regurgitation reduction device 1034 can be adapted to reduce regurgitation of any heart valve. For example, in Figures 14A and 14B the valved regurgitation reduction device 1034 is placed in the mitral valve MV where it is held in place by the anchor 24. Referring to Figure 14A, when the heart is in the diastolic phase, the valve 1000 opens and the mitral valve MV opens around the cage 900 and cover 902 of the valved regurgitation reduction device 1034. Blood flows from the left atrium LA to the left ventricle LV between the mitral valve MV and the cage 900 and cover 902 as indicated by arrows 2022 and/or through the valve 1000 as indicated by arrow 2024.

Referring to Figure 14B, when the heart is in the systolic phase, the valve 1000 closes and the mitral valve MV closes around the cage 900 and cover 902 of the valved regurgitation reduction device 1034. Blood flow from the left ventricle LV to the left atrium LA is blocked by the mitral valve MV closing on the cage 900 and cover 902 and by the valve 1000 being closed and blocking blood flow as indicated by arrow 2030.

The anchor 24 can take a wide variety of different forms. The anchor can be introduced transvascularly or surgically. A few non-limiting examples of the many possible configurations for the anchor 24 are disclosed herein. Other anchor configurations may be implemented without departing from the scope of the present application.

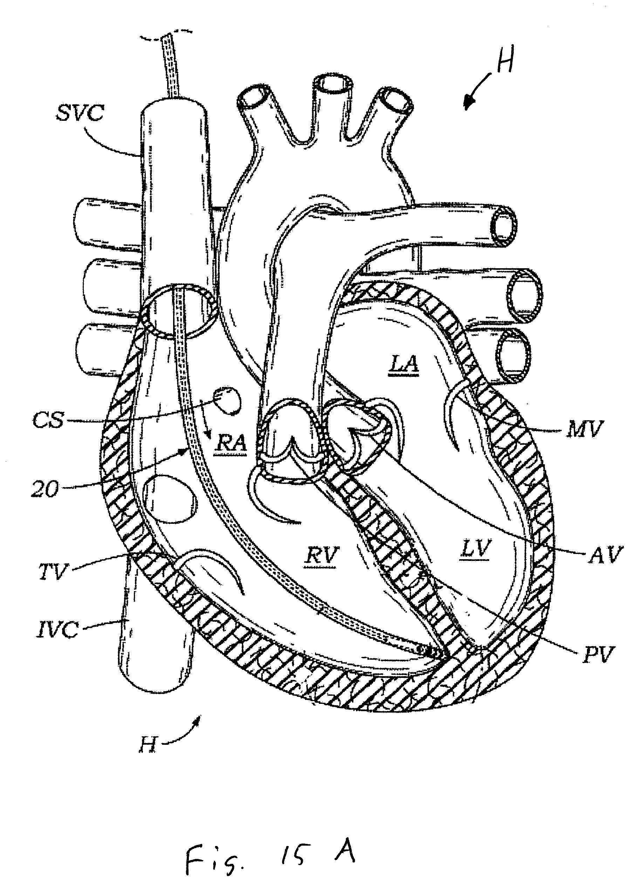

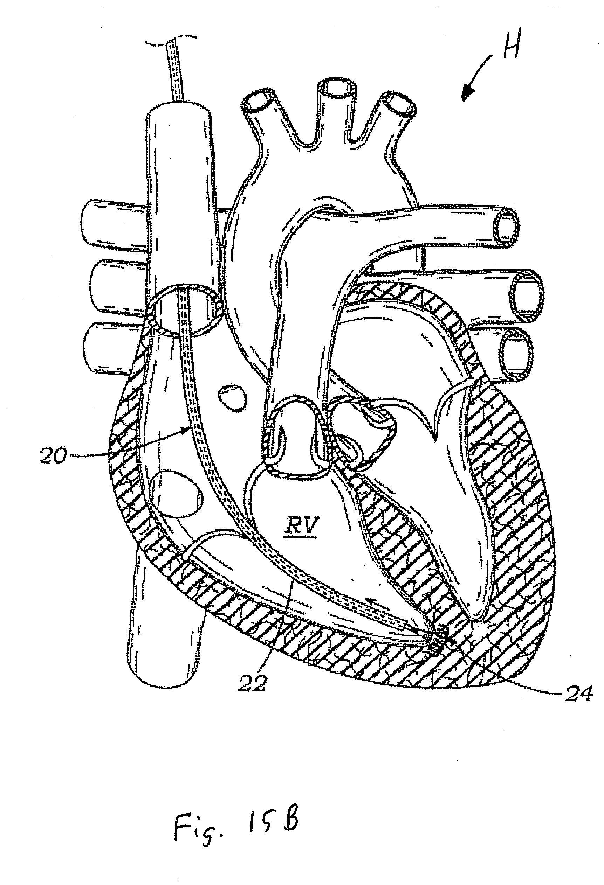

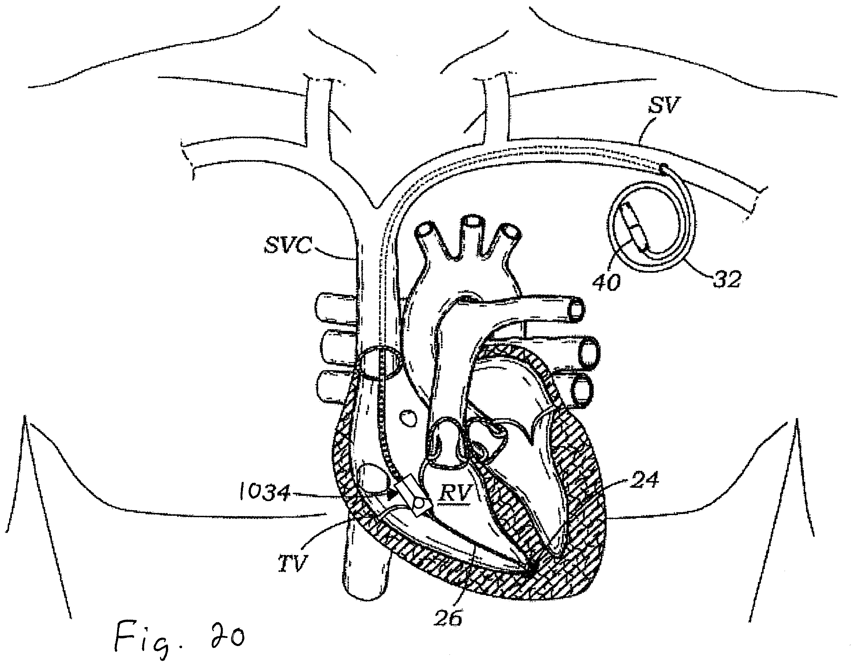

Figures 15A and 15B are taken from Published Patent Cooperation Treaty Application No. WO 2013/173587. Figures 15A and 15B show introduction of an anchoring catheter 20 into the right ventricle as a first step in deploying a valved regurgitation reduction device 1034 for reducing tricuspid valve regurgitation. The anchoring catheter 20 can enter the right atrium RA from the superior vena cava SVC after having been introduced to the subclavian vein (see Figure 20) using well-known methods, such as the Seldinger technique. Access for any of the devices disclosed herein may be femoral or subclavian. More particularly, the anchoring catheter 20 preferably tracks over a pre-installed guide wire (not shown) that has been inserted into the subclavian vein and steered through the vasculature until it resides at the apex of the right ventricle. The physician advances the anchoring catheter 20 along the guide wire until its distal tip is touching at or near the ventricular apex, as seen in Figure 15A.

Figure 15B shows retraction of a sheath 22 of the anchoring catheter 20 after installing a device anchor 24 at or near the apex of the right ventricle RV. The sheath 22 will generally be removed completely from the patient's body in favor of the anchoring catheter. The device anchor 24 is attached to an elongated anchor rail 26, which in some versions is constructed to have good capacity for torque. For instance, the anchor rail 26 may be constructed as a braided wire rod, or cable. The anchor 24 includes a plurality of circumferentially distributed and distally-directed sharp tines or barbs that pierce the tissue of the ventricular apex. The barbs 28 may be provided with an outward elastic bias so that they curl outward upon release from the sheath. Desirably the barbs are made of a super-elastic metal such as Nitinol. Although the particular device anchor 24 shown in Figures 15A and 15B is considered highly effective, other anchors are contemplated, such as shown and described below, and the application should not be considered limited to any particular type of anchor.

To facilitate central positioning of the anchor rail 26 during deployment the device may be implanted with the assistance of a fluoroscope. For example, a pigtail catheter may be placed in the right ventricle and contrast injected. This allows the user to see a clear outline of the annulus and the right ventricle. At this point, a frame of interest is selected (e.g., end systole) in which the annulus is clearly visible and the annulus to ventricular apex distance is minimized. On the monitor, the outline of the right ventricle, the annulus, and the pulmonary artery may be traced. The center of the annulus is then identified and a reference line placed 90° to it may be drawn extending to the right ventricular wall. This provides a clear linear target for anchoring. The anchor 24 is preferably located in the base of the ventricle between the septum and the free wall. Aligning the anchor rail 26 in this manner helps center the eventual positioning of a valved regurgitation reduction device 1034 of the system within the tricuspid leaflets.

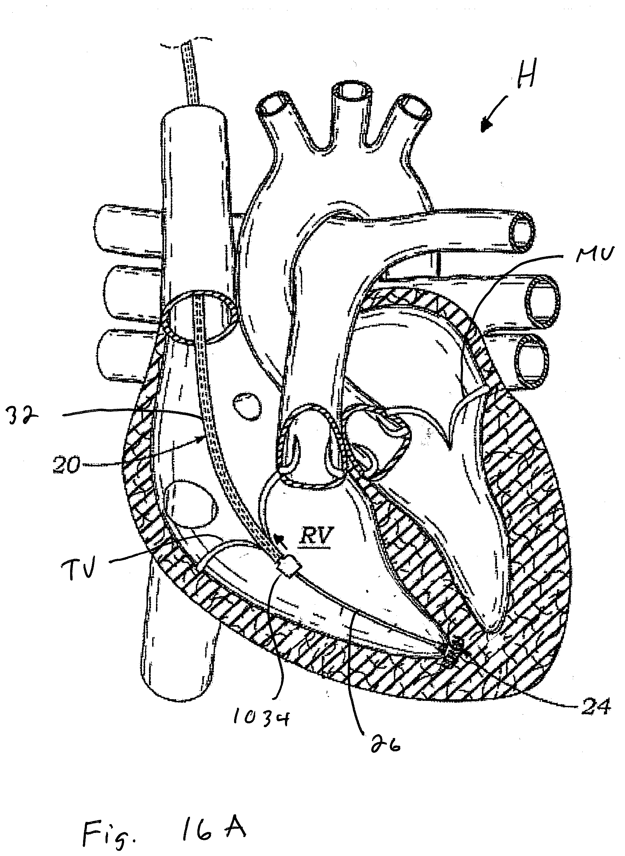

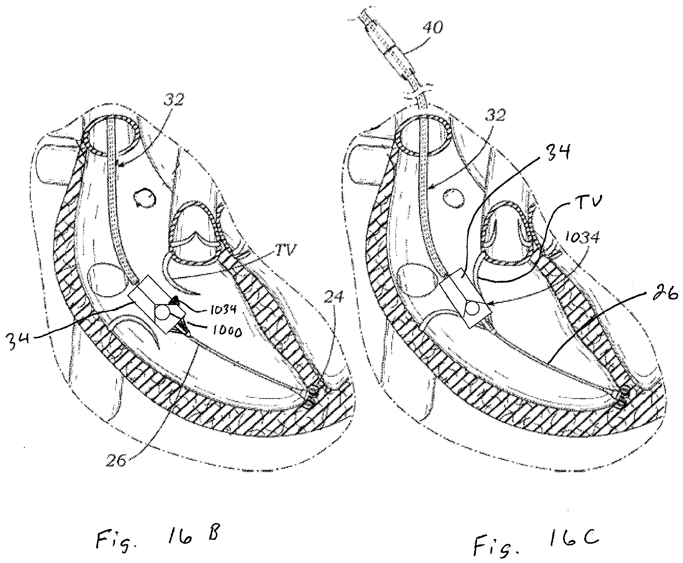

Figure 16A illustrates deployment of a valved regurgitation reduction device 1034 from a delivery catheter 32 that is disposed along the anchor rail 26. The valved regurgitation reduction device 1034 can be deployed from the delivery catheter 32 in the heart valve, such as the tricuspid valve TV or mitral valve. The deployed valved regurgitation reduction device 1034 can expand to the condition illustrated by Figures 16B and 16C or is expanded to the position illustrated by Figures 16B and 16C by an inflatable device.

In Figures 16A-16C, the valved regurgitation reduction device 1034 fastens to a distal end of the delivery catheter 32, both of which slide along the anchor rail 26, which has been previously positioned as described above. Ultimately, as seen in Figures 16B and 16C, the valved regurgitation reduction device 1034 resides within the tricuspid valve TV. Figure 16B illustrates the heart in the diastolic phase, with the leaflets of the tricuspid valve TV spaced apart from the valved regurgitation reduction device 1034. Figure 16C illustrates the heart in the systolic phase, with the leaflets closed in contact with the valved regurgitation reduction device 1034.

The delivery catheter 32 optionally remains in the body as seen in Figure 20, and the prefix "delivery" should not be considered to limit its function. A variety of valved regurgitation reduction devices 1034 are described herein, the common feature of which is providing a valved-plug of sorts within the heart valve leaflets to both mitigate or otherwise eliminate regurgitation in the systolic phase and enhance blood flow in the diastolic phase.

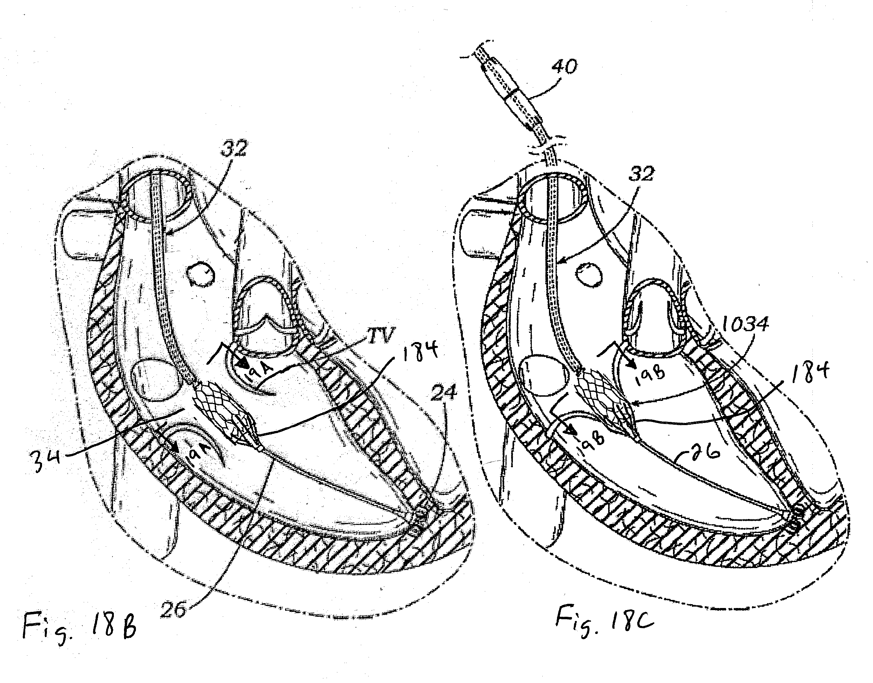

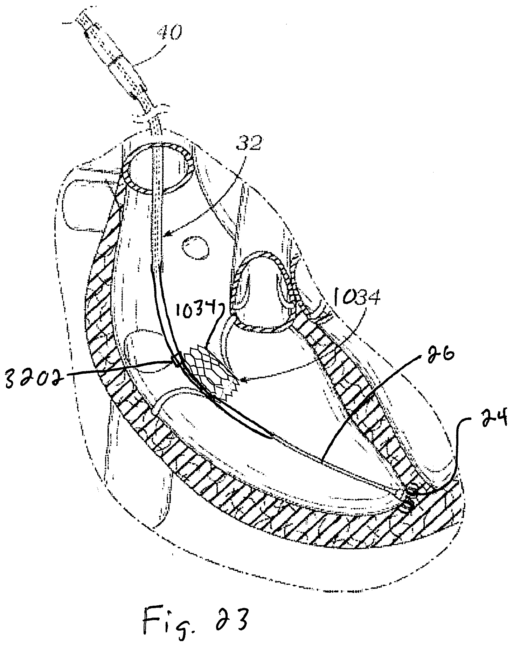

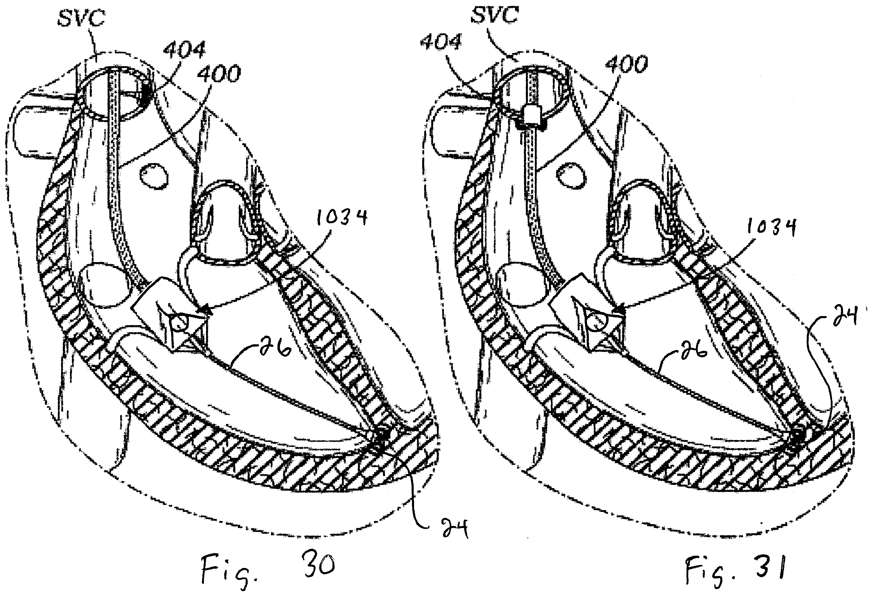

The valved regurgitation reduction device 1034 may be mounted to the anchor rail 26 and/or catheter 32 in a wide variety of different ways. For example, the valved regurgitation reduction device 1034 may be mounted to the anchor rail with a strut structure where the anchor rail passes through the valved regurgitation reduction device 1034 (See Figures 18B and 18C), by external wires where the anchor rail 26 does not pass through the valved regurgitation reduction device 1034 (See Figures 30A and 30B), and/or an outside surface of the valved regurgitation reduction device 1034 may be mounted to the anchor rail 26 and/or catheter 32 (See Figure 23).

A locking mechanism can be coupled to the valved regurgitation reduction device 1034 to lock its position within the tricuspid valve TV and relative to the fixed anchor rail 26. For example, a locking collet 40 along the length of the delivery catheter 32 permits the physician to selectively lock the position of the delivery catheter, and thus the connected valved regurgitation reduction device 1034, on the anchor rail 26. There are of course a number of ways to lock the valved regurgitation reduction device 1034 on the catheter and/or the guide rail, and the application should not be considered limited to what is illustrated. For instance, rather than a locking collet 40, a crimpable section such as a stainless steel tube may be included on the delivery catheter 32 at a location near the skin entry point and spaced apart from the location of the coapting element 34. The physician need only position the coapting element 34 within the leaflets, crimp the catheter 32 onto the anchor rail 26, and then sever both the catheter and rail above the crimp point.

In Figure 20, the delivery catheter 32 is left in place after placement of the valved regurgitation reduction device 1034. Alternatively, the delivery catheter is removed, leaving only the anchor 24 and the valved regurgitation reduction device 1034. In Figure 20, an entire regurgitation reduction system 30 can be seen extending from near the apex of the right ventricle RV upward through the superior vena cava SVC and into the subclavian vein SV. A proximal length of the delivery catheter 32 including the locking collet 40 exits the subclavian vein SV through a puncture and remains implanted subcutaneously; preferably coiling upon itself as shown. In the procedure, the physician first ensures proper positioning of the valved regurgitation reduction device 1034 within the tricuspid valve TV, then locks the delivery catheter 32 with respect to the anchor rail 26 by actuating the locking collet 40, and then severs that portion of the delivery catheter 32 that extends proximally from the locking collet. The collet 40 and/or coiled portion of the delivery catheter 32 may be sutured or otherwise anchored in place to subcutaneous tissues outside the subclavian vein SV. It is also worth noting that since the delivery catheter 32 slides with respect to the anchor rail 26, it may be completely removed to withdraw the valved regurgitation reduction device 1034 and abort the procedure - either during or after implantation. The implant configuration is similar to that practiced when securing a pacemaker with an electrode in the right atrium muscle tissue with the leads extending to the associated pulse generator placed outside the subclavian vein. Indeed, the procedure may be performed in conjunction with the implant of a pacing lead.

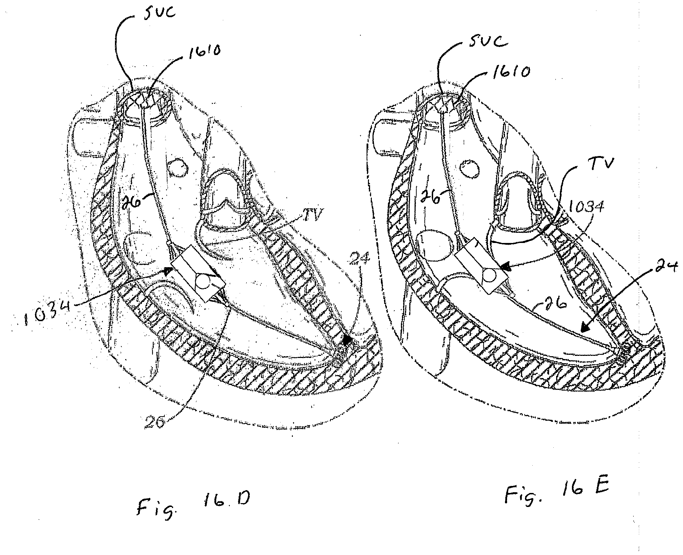

Figures 16D and 16E illustrate an example where the delivery catheter is removed, leaving only the anchor 24 and the valved regurgitation reduction device 1034. Figure 16D illustrates the heart in the diastolic phase, with the leaflets of the tricuspid valve TV spaced apart from the valved regurgitation reduction device 1034. Figure 16E illustrates the heart in the systolic phase, with the leaflets closed in contact with the valved regurgitation reduction device 1034. In the example illustrated by Figures 16D and 16E, the rail 26 is connected to a stent 1610 disposed in the superior vena cava SVC to set the position of the valved regurgitation reduction device 1034, with the catheter removed. However, the anchor can take a wide variety of different forms.

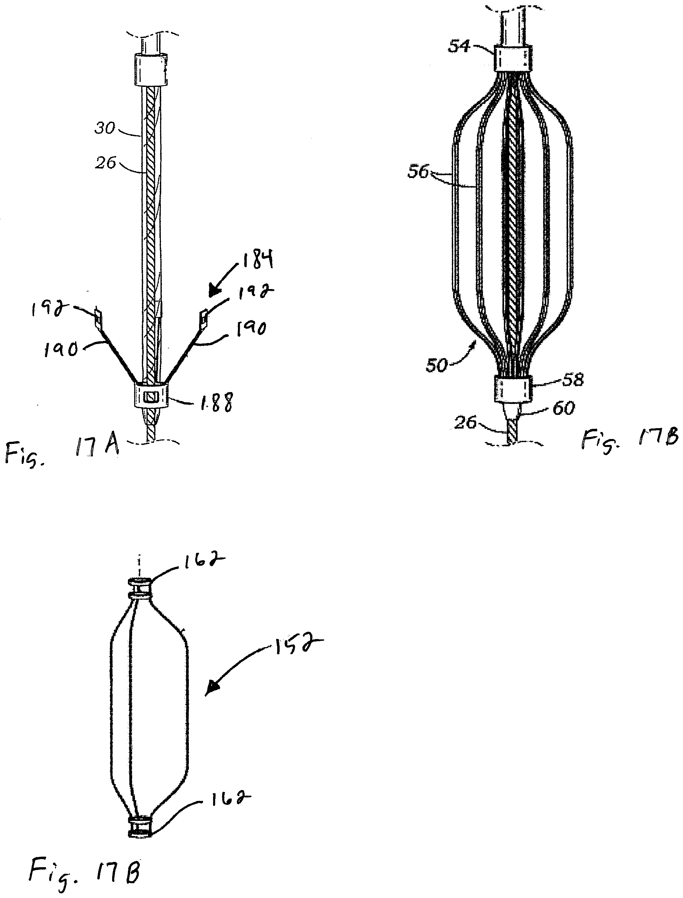

The valved regurgitation reduction device 1034 can be attached to the anchor rail 26 in a wide variety of different ways. Figures 17A-17C illustrate a few of the many possible structures that can be used to slideably couple the valved regurgitation reduction device 1034 to the rail. Referring to Figure 17A, a multi-strut frame 184 includes a collar 188 that slideably couples and is optionally securable to the rail 26. The collar 188 has a plurality of, preferably three, struts 190 that angle outward from it in a proximal or atrial direction and terminate in small pads or feet 192. The feet 192 attach to a distal end of the valved regurgitation reduction device 1034. The struts 190 may be resilient such that the feet 192 apply radial outward forces to the valved regurgitation reduction device 1034 so as to maintain the distal end of the valved regurgitation reduction device 1034 open.

Referring to Figure 17B, a three- strut mechanical frame 152 is retained by a pair of end collars 162 that are optionally secured to a delivery catheter 32 and/or slideably coupled and optionally securable to the rail 26. The frame 152 is compressible and expands in its relaxed configuration. Figure 17C illustrates an inner strut frame 50 that includes a short tubular collar 54 that optionally fastens to the distal end of the delivery catheter 32 and/or which slideably couples and is optionally securable to the rail 26. A second tubular collar 58 holds together the distal ends of the struts 56 and attaches to a small ferrule 60 having a through bore that slides over the anchor rail 26. The second collar 58 and/or the small ferrule 60 slideably couple and are optionally securable to the rail 26. Each of the struts 56 has proximal and distal ends that are formed as a part of (or constrained within) these collars 54, 58 and a mid-portion that arcs radially outward to extend substantially parallel to the axis of the valved regurgitation reduction device 1034. The frame shape is thus a generally elongated oval. In the illustrated example, there are six struts 56 in the frame 50, although more or less could be provided. The struts 56 are desirably formed of a super-elastic material such as Nitinol so as to have a minimum amount of rigidity to form the generally cylindrical outline of the frame but maximum flexibility so that the frame deforms from the inward forces imparted by the heart valve leaflets.

A number of different valved regurgitation reduction devices 1034 are described in the present application. Indeed, the present application provides a plurality of solutions for preventing regurgitation in atrioventricular valves, none of which should be viewed as necessarily more effective than another. For example, the choice of valved regurgitation reduction device 1034 may depend partly on physician preference, partly on anatomical particularities, partly on the results of clinical examination of the condition of the patient, and other factors.

Referring to Figures 6-8 and 18A-18E, the valved regurgitation reduction device 1034 can comprise a valve 1000 made at least partially from bioprosthetic tissue disposed within an expandable and contractible frame 900 that is at least partially covered 902 with bioprosthetic tissue. The frame 900 may be rigid after being expanded from the condition illustrated by Figure 6 to the condition illustrated by Figure 7. The bioprosthetic tissue covering 902 helps reduce material interactions between the native leaflets and the inner mechanical frame. As mentioned above, the regurgitation reduction device 1034 can be effectively deployed at either the tricuspid or the mitral valve. The former typically has three leaflet cusps defined around the orifice, the latter just two. The tissue-covered mechanical frame structure thus represents an effective co-optation element for both valves.

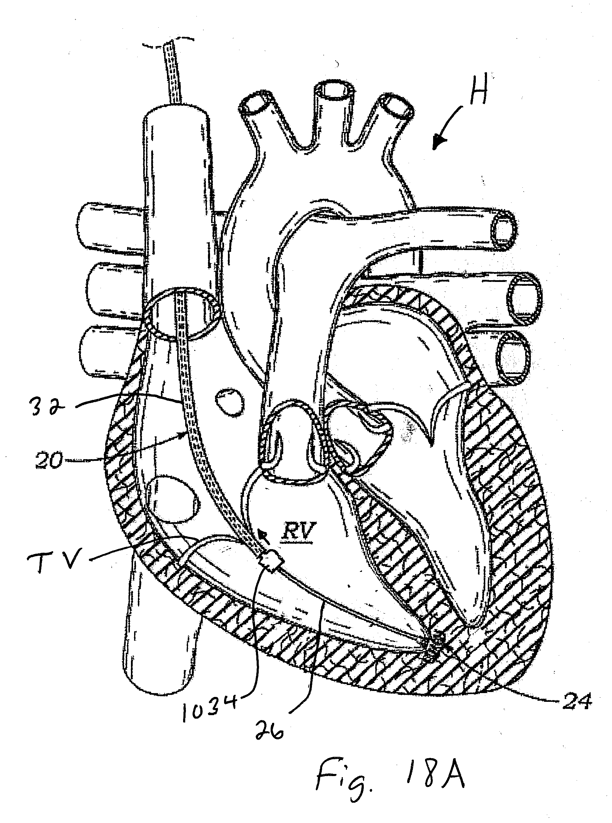

Figure 18A illustrates deployment of a valved regurgitation reduction device 1034 from a delivery catheter 32 that is disposed along the anchor rail 26. The valved regurgitation reduction device 1034 can be deployed from the delivery catheter 32 in the heart valve, such as the tricuspid valve TV or mitral valve MV. The deployed valved regurgitation reduction device 1034 expands to the condition illustrated by Figures 18B and 18C or is expanded to the position illustrated by Figures 18B and 18C by an inflatable device.

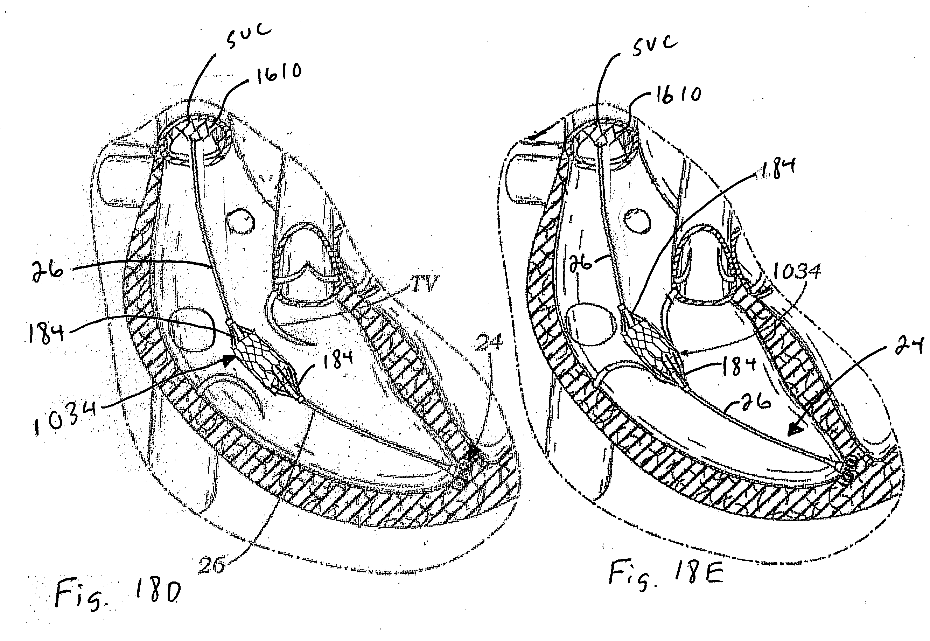

In the example illustrated by Figures 18B and 18C, the valved regurgitation reduction device 1034 illustrated by Figures 6-8 has a distal end mounted to the frame 184 illustrated by Figure 17A to slideably couple it to the rail 26. In the example illustrated by Figures 18D and 18E, the valved regurgitation reduction device 1034 illustrated by Figures 6-8 has both ends mounted to a frame 184 illustrated by Figure 17A to slideably couple it to the rail 26.

Figures 18A-18C illustrate deployment of a delivery catheter 32 advanced along the anchor rail 26 to position the valved regurgitation reduction device 1034 within the tricuspid valve TV. The valved regurgitation reduction device 1034 optionally fastens to a distal end of the delivery catheter 32, both of which slide along the anchor rail 26, which has been previously positioned as described above. Ultimately, as seen in Figures 18B and 18C, the valved regurgitation reduction device 1034 resides within the tricuspid valve TV. Figure 18B illustrates the heart in the diastolic phase, with the leaflets of the tricuspid valve TV spaced apart from the valved regurgitation reduction device 1034. Figure 18C illustrates the heart in the systolic phase, with the leaflets closed in contact with the valved regurgitation reduction device 1034.

The delivery catheter 32 optionally remains in the body as seen in Figure 20. Alternatively, the frame 184 is connectable to the rail 26, the proximal end of the rail is connectable to another structure in the heart or body, and the delivery catheter 32 is removed from the body. This leaves only the valved regurgitation reduction device 1034 and an anchor 24.

A locking mechanism is provided to lock the valved regurgitation reduction device 1034 in its position within the tricuspid valve TV and relative to the fixed anchor rail 26. For example, a locking collet 40 along the length of the delivery catheter 32 and/or providing the frame 184 with a mechanism that is selectively lockable to the rail 26 permits the physician to selectively lock the position of the delivery catheter and/or the connected valved regurgitation reduction device 1034, on the anchor rail 26. There are of course a number of ways to lock the valved regurgitation reduction device 1034, the catheter and/or the guide rail, and the application should not be considered limited to what is illustrated.

Figures 18D and 18E illustrate an example, where the delivery catheter 32 of Figures 18A-18C is removed, leaving only the anchor 24 and the valved regurgitation reduction device 1034. Figure 18D illustrates the heart in the diastolic phase, with the leaflets of the tricuspid valve TV spaced apart from the valved regurgitation reduction device 1034. Figure 18E illustrates the heart in the systolic phase, with the leaflets closed in contact with the valved regurgitation reduction device 1034. In the example illustrated by Figures 18D and 18E, the rail 26 is connected to a stent 1610 disposed in the superior vena cava SVC to set the position of the valved regurgitation reduction device 1034, with the catheter removed. However, the anchor 24 can take a wide variety of different forms.

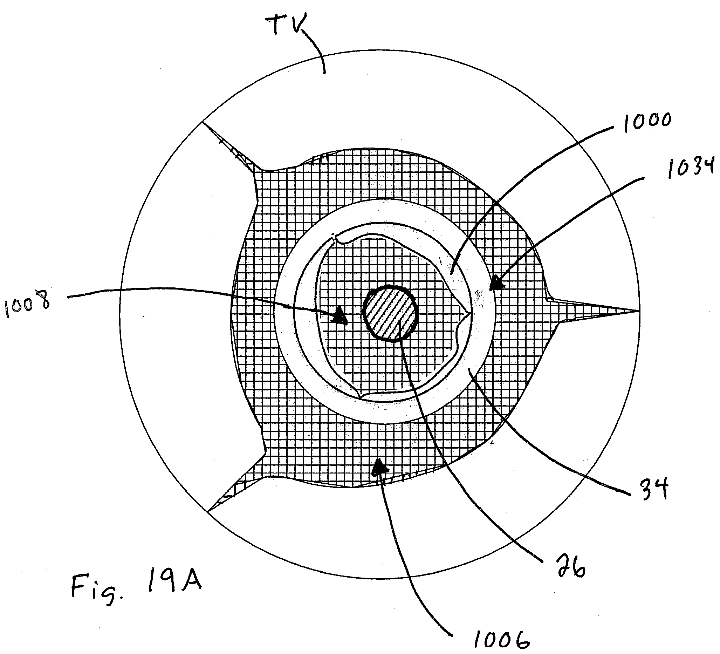

Figure 19A illustrates that when the heart is in the diastolic phase, the valve 1000 opens and the tricuspid valve TV opens around the cage 900 and cover 902 of the valved regurgitation reduction device 1034. The rail 26 is disposed inside the open valve 1000. Blood flows from the right atrium RA to the right ventricle RV between the tricuspid valve TV and the cage 900 and cover 902 as and through the valve 1000 around the rail 26.

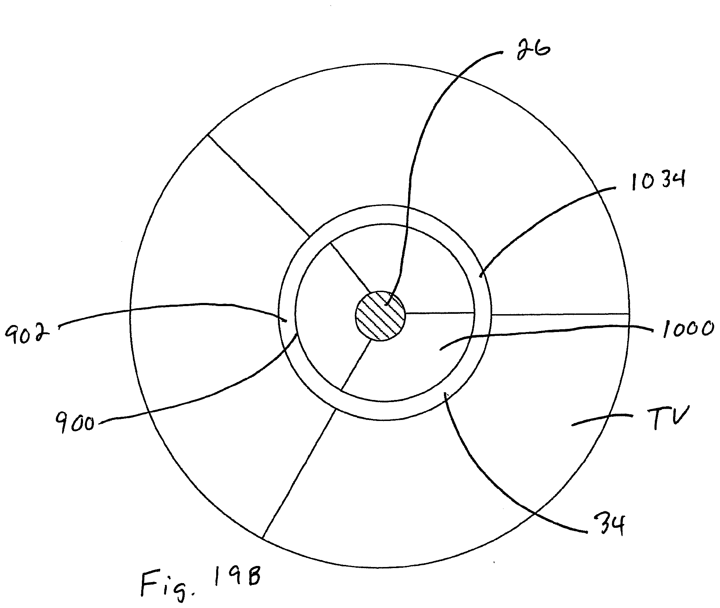

Referring to Figure 19B, when the heart is in the systolic phase, the valve 1000 closes around the rail 26 and the tricuspid valve TV closes around the cage 900 and cover 902 of the valved regurgitation reduction device 1034. The leaflets or cusps of the valve 1000 seal against one another and against the rail 26. The rail 26 or the portion of the rail that engages the valve 1000 can be covered, coated, or made from a material that is compatible with the valve. For example, the rail 26 or the portion of the rail that engages the valve 1000 may be covered, coated, or made from the same material as the leaflets of the valve. Blood flow from the right ventricle RV to the right atrium RA is blocked by the tricuspid valve TV closing on the cage 900 and cover 902 and by the valve 1000 being closed against the rail 26.

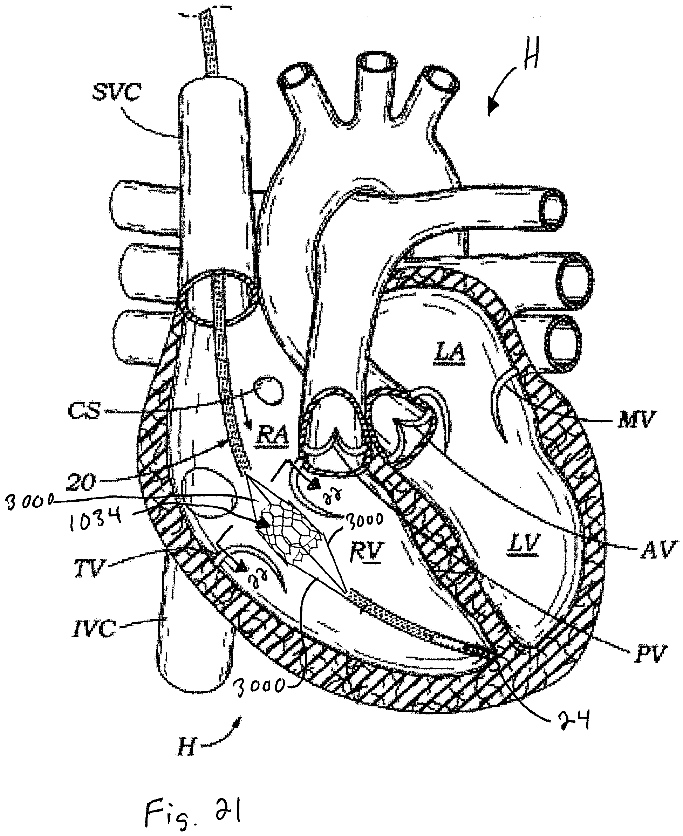

In the example illustrated by Figure 21, the valved regurgitation reduction device 1034 illustrated by Figures 6-8 is mounted to wires 3000 that are part of the rail 26 and/or are disposed inside the delivery catheter 32. The wires 3000, rail 26, and /or delivery catheter 32 are adjustable to adjust the position and orientation of the valved regurgitation reduction device 1034 with respect to the tricuspid valve TV (or mitral valve MV). For example, extending or retracting one or more, but less than all of the wires 3000 pivots the valved regurgitation reduction device 1034 to allow for axial alignment of the valved regurgitation reduction device 1034 with respect to the tricuspid valve TV (or mitral valve MV).

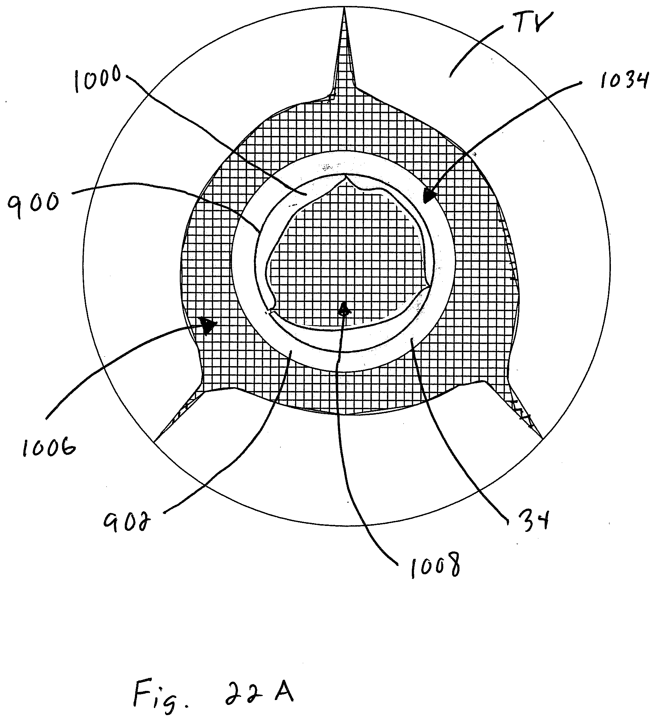

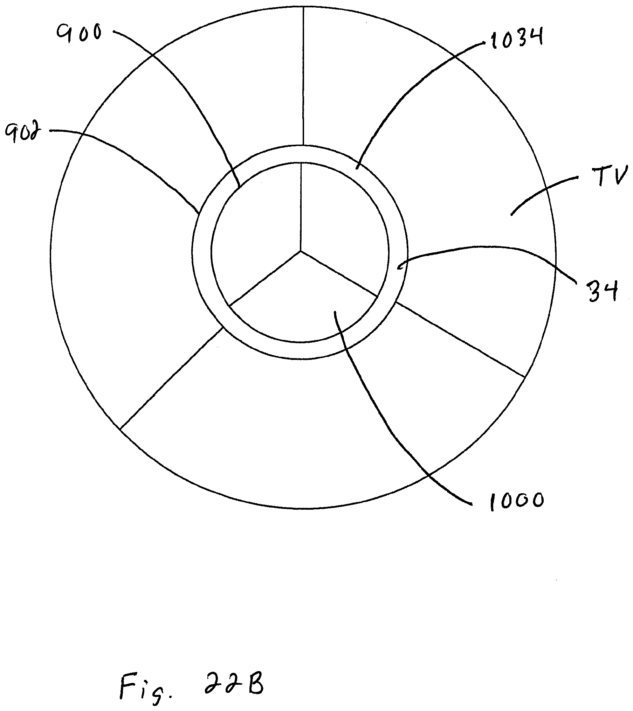

Figure 22A illustrates that when the heart is in the diastolic phase, the valve 1000 opens and the tricuspid valve TV opens around the cage 900 and cover 902 of the valved regurgitation reduction device 1034. No rail 26 is disposed inside the open valve 1000. Blood flows from the right atrium RA to the right ventricle RV between the tricuspid valve TV and the cage 900 and cover 902 and through the valve 1000. Referring to Figure 22B, when the heart is in the systolic phase, the valve 1000 closes and the tricuspid valve TV closes around the cage 900 and cover 902 of the valved regurgitation reduction device 1034. The leaflets or cusps of the valve 1000 seal against one another. Blood flow from the right ventricle RV to the right atrium RA is blocked by the tricuspid valve TV closing on the cage 900 and cover 902 and by the valve 1000 being closed.

In the example illustrated by Figure 23, an external surface of the valved regurgitation reduction device 1034 illustrated by Figures 6-8 is attached to the rail 26 and/or the catheter 32. For example, the valved regurgitation reduction device 1034 can be connected to a distal end of the catheter 32 and/or an outside surface of the valved regurgitation reduction device 1034 can include a connection 3202 that is slideable on the rail 26 until the valved regurgitation reduction device 1034 is positioned in the tricuspid valve TV (or mitral valve MV) and is then secured to the rail 26 to set the position of the valved regurgitation reduction device 1034 relative to the tricuspid valve TV (or mitral valve MV) The valve 1000 and tricuspid valve TV (or mitral valve MV) in the arrangement illustrated by Figure 23 open and close in the same manner as in the arrangement illustrated by Figure 21 (See Figures 22A and 22B).

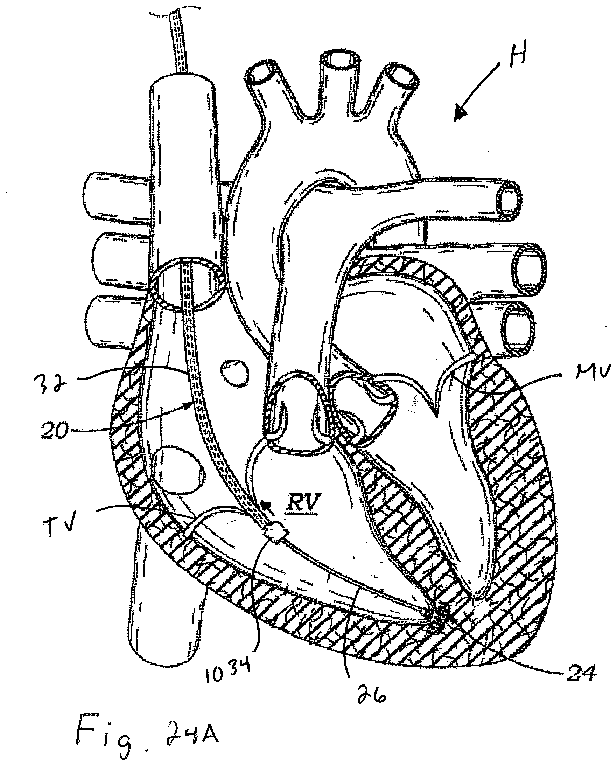

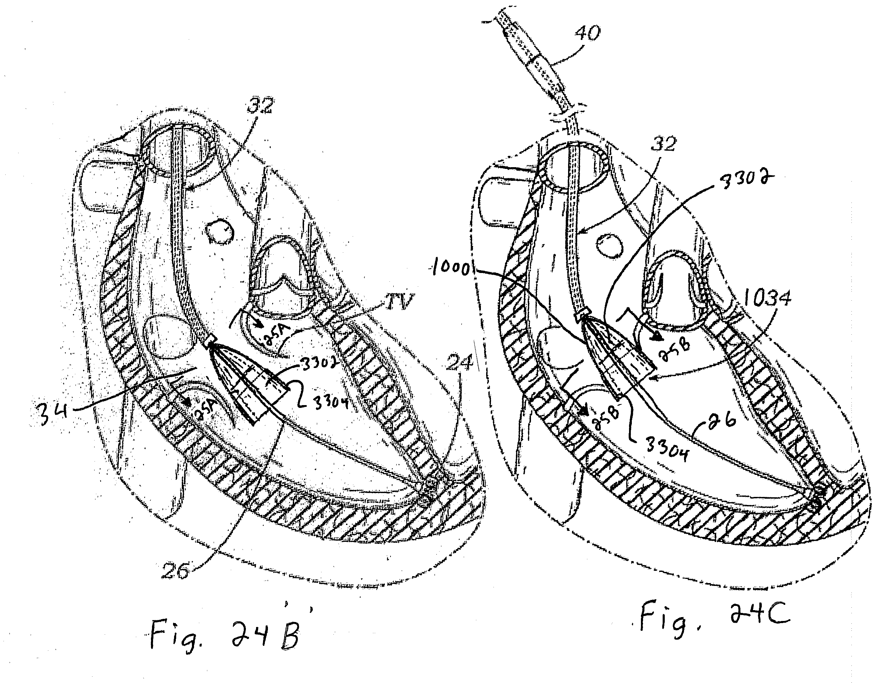

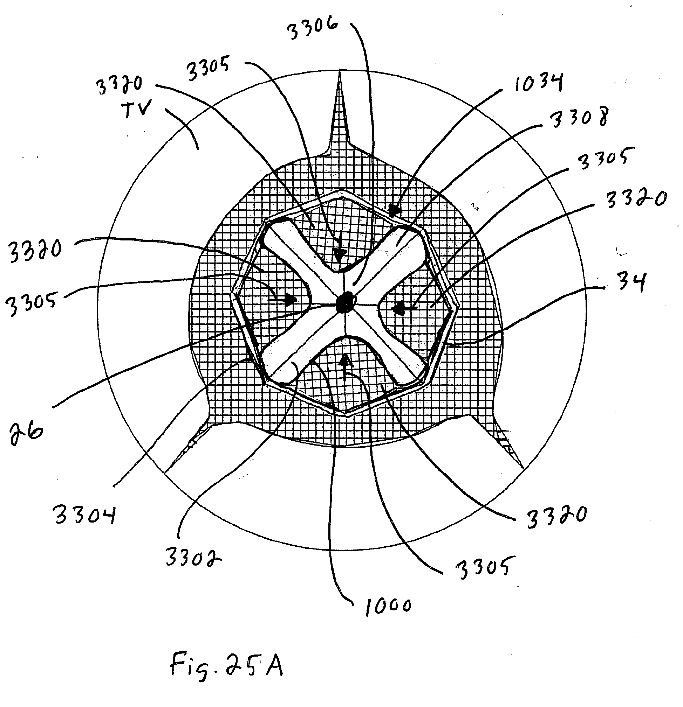

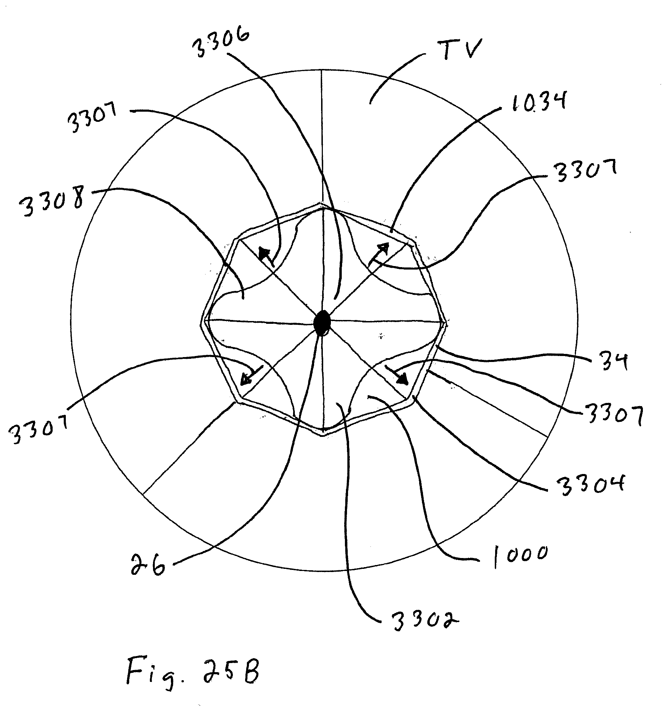

Figures 24A-24C, 25A, and 25B illustrate an example where the valve 1000 of the valved regurgitation reduction device 1034 includes a sealing element 3302 and an outer skirt or ring 3304. The sealing element 3302 includes a substantially stationary center portion 3306 and radially outer portion 3308. The radially outer portion 3308 moves inward (see arrow 3305 in Figure 25A) to open and radially outward to close (see arrow 3307 in Figure 25B). In the example illustrated by Figures 24A-24C, 25A and 25B, the radially outer portion 3308 seals against the outer skirt or ring 3304. The valve 1000 illustrated by Figures 24A-24C, 25A and 25B is expandable so that it can be installed transvascularly. The valve 1000 illustrated by Figures 24A, 24B, 25A and 25B is constructed substantially as shown and described in US Patent No. 6,540,782.

Figure 24A illustrates deployment of a valved regurgitation reduction device 1034 from a delivery catheter 32 that is disposed along the anchor rail 26. The valved regurgitation reduction device 1034 can be deployed from the delivery catheter 32 in the heart valve, such as the tricuspid valve TV or mitral valve MV. The deployed valved regurgitation reduction device 1034 expands to the condition illustrated by Figures 24B and 24C or is expanded to the position illustrated by Figures 24B and 24C by an inflatable device.

In the example illustrated by Figures 24A-24C, the rail 26 extends through center portion 3306 of the valve 1000 of the valved regurgitation reduction device 1034 to slideably couple the valved regurgitation reduction device 1034 to the rail 26. Figures 24A-24C illustrate deployment of a delivery catheter 32 advanced along the anchor rail 26 to position the valved regurgitation reduction device 1034 within the tricuspid valve TV. The center portion 3308 of the valved regurgitation reduction device 1034 optionally fastens to a distal end of the delivery catheter 32, both of which slide along the anchor rail 26, which has been positioned. Ultimately, as seen in Figures 24B and 24C, the valved regurgitation reduction device 1034 resides within the tricuspid valve TV. Figure 24B illustrates the heart in the diastolic phase, with the leaflets of the tricuspid valve TV spaced apart from the valved regurgitation reduction device 1034. Figure 24C illustrates the heart in the systolic phase, with the leaflets closed in contact with the valved regurgitation reduction device 1034.

The delivery catheter 32 optionally remains in the body as seen in Figure 20. Alternatively, the center portion 3308 of the valve 1000 is connectable to the rail 26, the proximal end of the rail is connectable to another structure in the heart or body, and the delivery catheter 32 is removed from the body. This leaves only the valved regurgitation reduction device 1034 and an anchor 24.

A locking mechanism is provided on the valved regurgitation reduction device 1034 to lock its position within the tricuspid valve TV and relative to the fixed anchor rail 26. For example, a locking collet 40 along the length of the delivery catheter 32 and/or providing the center portion 3306 of the valve 1000 with a mechanism that is selectively lockable to the rail 26 permits the physician to selectively lock the position of the delivery catheter and/or the connected valved regurgitation reduction device 1034, on the anchor rail 26. There are of course a number of ways to lock the valved regurgitation reduction device 1034, the catheter 32 and/or the guide rail 26, and the application should not be considered limited to what is illustrated.

Figure 25A illustrates that when the heart is in the diastolic phase, the valve 1000 opens and the tricuspid valve TV opens around the valved regurgitation reduction device 1034. The radially outer portion 3308 moves inward 3305 away from the outer skirt 3304 to open. The blood flows through gaps 3320 between the outer skirt 3304 and the sealing element 3302. The rail 26 is disposed inside the open valve 1000, but not in the gaps 3320. Blood flows from the right atrium RA to the right ventricle RV between the tricuspid valve TV and the valved regurgitation reduction device 1034 and through the valve 1000 around the rail 26.

Referring to Figure 25B, when the heart is in the systolic phase, the valve 1000 closes by movement (indicated by arrows 33307) of the valve element 3302 into contact with the skirt 3304 and the tricuspid valve TV closes around the valved regurgitation reduction device 1034. In Figure 25B, there is no need to cover the rail 26 with a material that is compatible with the valve, since the moveable valve element does not engage the rail 26. Blood flow from the right ventricle RV to the right atrium RA is blocked by the tricuspid valve TV closing on valved regurgitation reduction device 1034 and by the valve 1000 being closed.

### Anchors and Alternative Anchor Placement

The anchor 24 can take a wide variety of different forms. The following description provides non-limiting examples of catheter, railing, and anchoring systems.

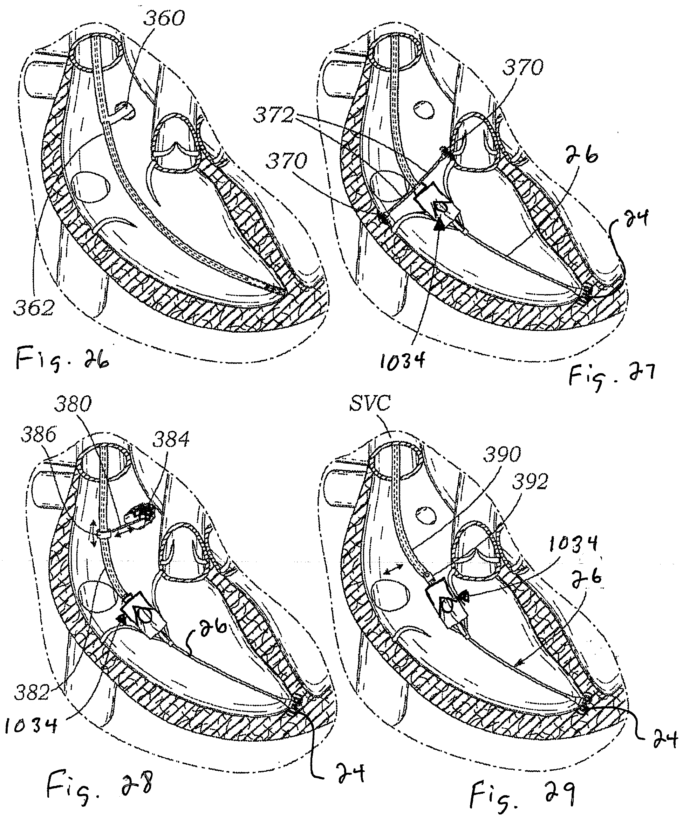

In the example illustrated by Figure 26 an anchoring catheter 360 is directed to or near the apex of the right ventricle using an L-shaped stabilizing catheter 362 secured within a coronary sinus. This configuration addresses the challenge of guiding the anchor delivery. The catheter 362 is capable of deflecting into an L-shape, and would be advanced from the SVC, into the right atrium, then into the coronary sinus, which would provide a stabilizing feature for the guide catheter. The catheter 362 could be maneuvered further in or out of the coronary sinus such that the "elbow" of the L-shape is positioned directly above the center of the valve, then the anchor catheter 360 could be delivered through the lumen of the guide catheter 362 and out a port at the elbow of the L-shape. A temporary stiffening "stylet" (not shown) could be used through the anchor rail lumen to ensure the anchor is delivered directly downwards to the ideal point at the RV apex.

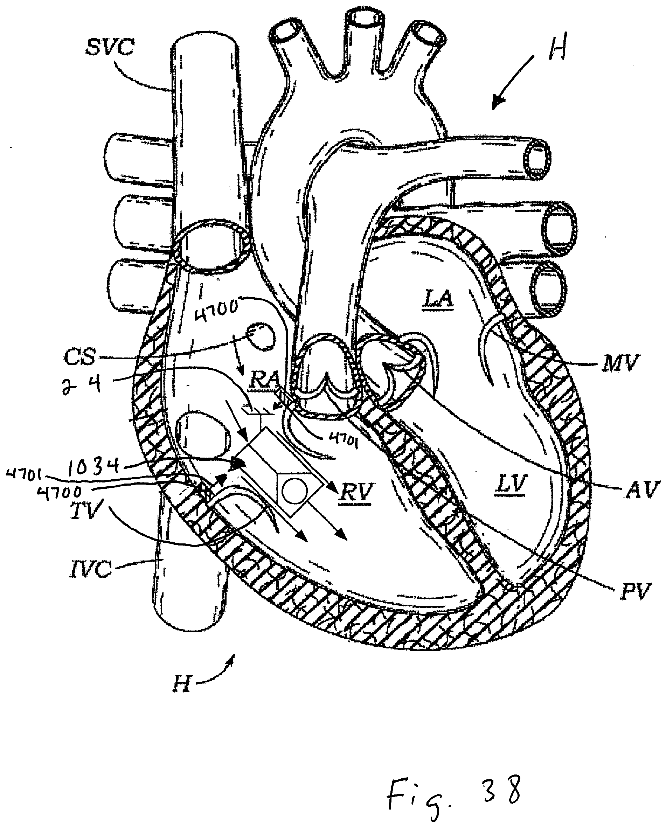

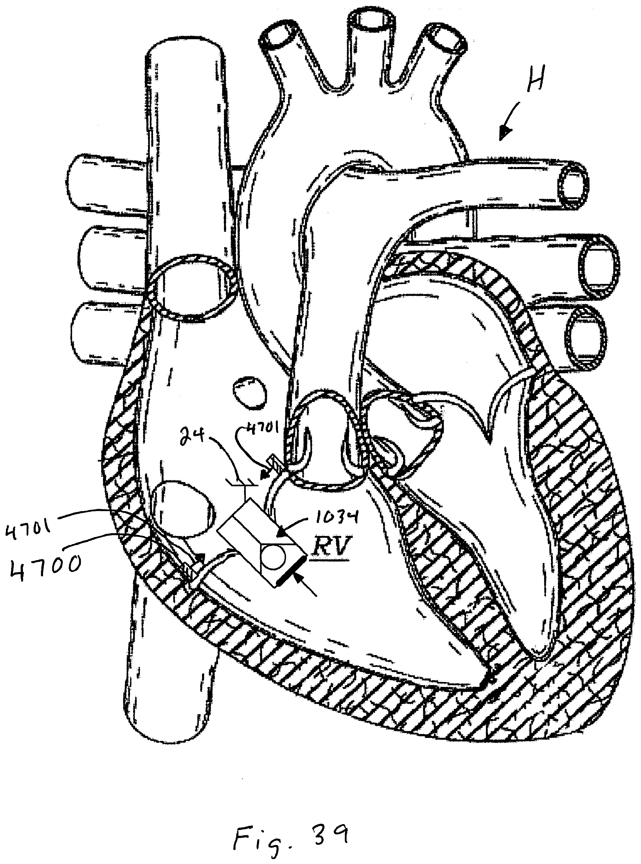

If any of the previously described anchoring options involving any combination of the RV, SVC, and IVC prove to be undesirable, the coapting element could instead be anchored directly to the annulus or a ring 4700 that is connected to the annulus (see Figures 38-43). As shown in Figure 27, a series of at least two anchors 370 could be deployed into the fibrous portion of the annulus, then cables or stabilizing rods 372 could be used to hang or suspend the valved regurgitation reduction device 1034 within the annulus plane. The ring 4700 illustrated by Figures 38-43 could be used to hang or suspend the valved regurgitation reduction device 1034 in the same or a similar way. Each support cable or rod 372 would need to be relatively taut, so as to prevent motion of the device towards the atrium during systole. Any number of support struts could be utilized. The support cables for suspending the valved regurgitation reduction device 1034 from the annulus could be relatively flexible, and thus the position and mobility of the device would be altered via tension in the cables. Alternatively, the support elements could be relatively stiff to decrease device motion, but this would require changing anchor position to reposition the coapting element. Although an anchor 376 to the RV apex is shown, the dual annulus anchors 370 might obviate the need for a ventricular anchor.

Figure 28 illustrates an example where an adjustable stabilizing rod 380 is mounted on a delivery catheter 382 and secured to an anchor 384 within the coronary sinus. The stabilizing rod 380 attaches via an adjustable sleeve 386 to the catheter 382, thus suspending the attached valved regurgitation reduction device 1034 down into the tricuspid valve TV. A sliding mechanism on the adjustable sleeve 386 permits adjustment of the length between the coronary sinus anchor 384 and the valved regurgitation reduction device 1034, thus allowing positioning of the coapting element at the ideal location within the valve plane. For further stability, this coronary sinus anchoring concept could also be coupled with a traditional anchor in the RV apex, as shown.

While venous access to the RV through the subclavian vein and into the superior vena cava is a routine procedure with minimal risk for complications, the fairly flat access angle of the SVC with respect to the tricuspid valve plane presents a number of challenges for proper orientation of the present valved regurgitation reduction device 1034 within the valve. If the catheter were not flexible enough to achieve the correct angle of the valved regurgitation reduction device 1034 with respect to the valve plane by purely passive bending, a flex point could be added to the catheter directly proximal to the coapting element via a pull wire attached to a proximal handle through a double lumen extrusion. For instance, Figure 29 illustrates an alternative delivery catheter 390 having a pivot joint 392 just above the valved regurgitation reduction device 1034 for angle adjustment. If a given combination of SVC access angle and/or RV anchor position resulted in a crooked valved regurgitation reduction device 1034 within the valve plane, the catheter 390 could be articulated using the pull wire (not shown) until proper alignment is achieved based on feedback from fluoroscopic views.

Additional flex points could be added to further facilitate control of device angle, e.g. another flex point could be added distal to the valved regurgitation reduction device 1034 to compensate for the possible case that the RV wall angle (and thus the anchor angle) is skewed with respect to the valve plane. This would require an additional independent lumen within the catheter body 390 to facilitate translation of another pull wire to operate the second flex feature. Alternatively, if a single flex point proximal to the valved regurgitation reduction device 1034 were determined to be sufficient for orienting the device, and if the catheter were rigid enough to resist the forces of systolic flow, the section 396 of the device distal to the coapting element could be removed all together. This would leave only one anchoring point for the device in the SVC or subcutaneously to the subclavian vein. Also, as an alternative to an actively-controlled flex point, the catheter could contain a shape-set shaft comprised of Nitinol or another shape memory material, which would be released from a rigid delivery sheath into its "shaped" form in order to optimize device angle from the SVC. It could be possible to have a few catheter options of varying pre-set angles, yet choose only one after evaluation of the SVC-to-valve plane angle via angiographic images.

Instead of using an active mechanism within the catheter itself to change its angle, another example takes advantage of the surrounding anatomy, i.e. the SVC wall. Figures 30 and 31 show two ways to anchor the delivery catheter 400 to the superior vena cava SVC for stabilizing a valved regurgitation reduction device 1034. For example, a variety of hooks or anchors 404 could extend from a second lumen within the catheter 402 with the ability to grab onto the SVC wall and pull the catheter in that direction. Alternatively, a stiffer element could extend outwards perpendicular to the catheter axis to butt up against the SVC wall and push the catheter in the opposite direction. For especially challenging SVC geometries, such a mechanism could potentially be useful for achieving better coaxial alignment with the valve.

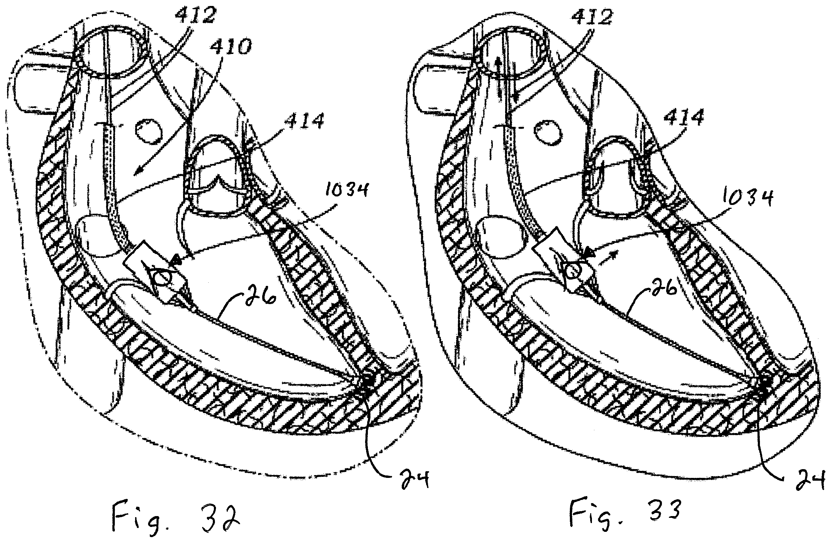

Figures 32 and 33 show an example with pull wires 412 extending through the delivery catheter 414 for altering the position of the valved regurgitation reduction device 1034 within the tricuspid valve leaflets. If the valved regurgitation reduction device 1034 is located out of the middle of the valve leaflets such that it does not effectively plug any regurgitant jets, which can be seen on echocardiography, then one of the pull wires 412 can be shortened or lengthened in conjunction with rotating the catheter 414 to reposition the valved regurgitation reduction device 1034.

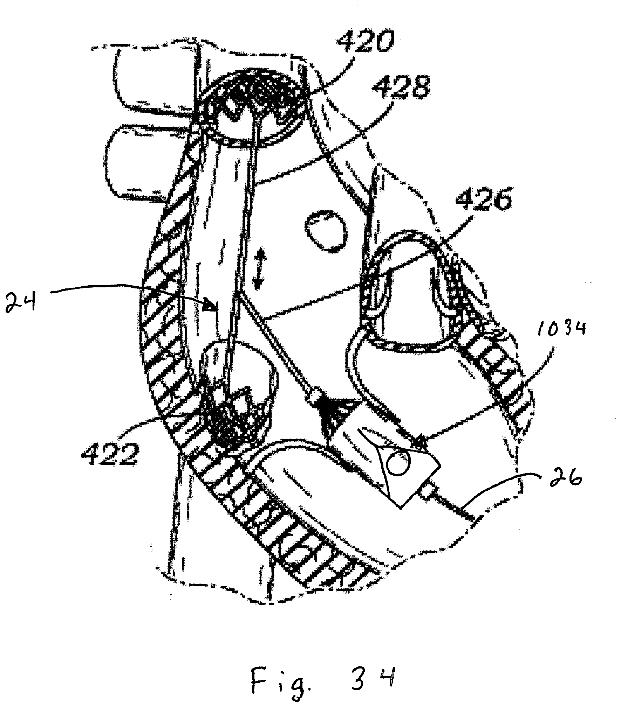

Although pacemaker leads are frequently anchored in the right ventricle with chronic success, the anchor for the present device would see significantly higher cyclic loads due to systolic pressure acting on the valved regurgitation reduction device 1034. Given that the right ventricle wall can be as thin as two millimeters near the apex and the tissue is often highly friable in patients with heart disease, anchoring a device in the ventricle may not be ideal. An alternative anchoring approach could take advantage of the fairy collinear orientation of the superior and inferior vena cava, wherein, as seen in Figure 34, two stent structures 420, 422 would effectively "straddle" the tricuspid valve by expanding one in the superior vena cava and the other in the inferior vena cava. The valved regurgitation reduction device 1034 would then hang down through the tricuspid valve plane from an atrial shaft 426 attached to a connecting wire or rod 428 between the two caval stents 420, 422. In order to resist motion of the valved regurgitation reduction device 1034 under systolic forces, the shaft 426 from which the coapting element 424 hangs would be fairly rigid under compressive and bending stresses. The valved regurgitation reduction device 1034 would desirably be positioned within the tricuspid valve TV using a sliding mechanism along the connecting rod 428 between the two caval stents.

The coaxial orientation of the SVC and IVC could also be leveraged for delivering an anchor into the RV. A delivery catheter could be passed through the SVC into the IVC, and a "port" or hole off the side of the delivery catheter could be aligned with the center of the valve. At this point, the anchor could be passed through the lumen of the delivery system and out the port, resulting in a direct shot through the center of the annulus and to the RV wall in the ideal central anchor location.

This concept could potentially be applied to the left side of the heart as well, to address mitral regurgitation. A valved regurgitation reduction device 1034 could reside between the mitral valve leaflets with anchors on both the proximal and distal ends: one attaching to the septal wall, and the other anchoring in the left atrial appendage. The septal anchor could be a helical or hook-style anchor, whereas the left atrial appendage anchor could be an expandable metallic structure with a plurality of struts or wireforms designed to oppose against the appendage wall and provide stability to the coapting element.

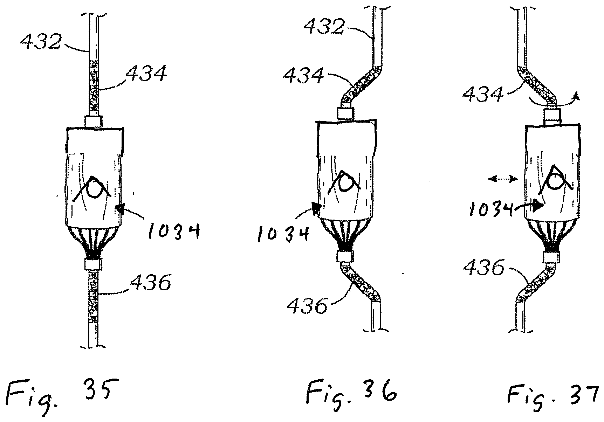

Figures 35-37 are schematic views of a valved regurgitation reduction device 1034 mounted for lateral movement on a flexible delivery catheter 432 that features controlled buckling. It is challenging to reposition the valved regurgitation reduction device 1034 from an off-center location to the ideal central location within the valve plane, given a fixed angle from the SVC and a fixed anchor position in the RV. The device catheter 432 could be comprised of a fairly stiff shaft except for two relatively flexible regions 434, 436 directly proximal and distal to the coapting element section. The farthest distal section of the valved regurgitation reduction device 1034 could be locked down relative to the anchor rail over which it slides, and then the catheter 432 could be advanced distally thus compressing it and causing the two flexible sections 434, 436 to buckle outwards and displace the valved regurgitation reduction device 1034 laterally with respect to the catheter axis (see Figure 36). Referring to Figure 37, the user could employ a combination of sliding and rotating of the catheter to reposition the valved regurgitation reduction device 1034 within the valve. Instead of locking the distal end of the catheter onto an anchor rail before adjustment, if the catheter were comprised of multiple lumens, the outer lumen could slide distally relative to the inner lumen, thus producing the same buckling effect.

Figures 38-43 illustrate an embodiment where the size of the valve annulus 300 is contracted or reduced in size as indicated by arrows 4701 before introduction of a valved regurgitation reduction device 1034 (or a coapting element disclosed by Published Patent Cooperation Treaty Application No. WO 2013/173587). By retracting the valve annulus 300, the regurgitation through the tricuspid valve TV (or other valve, such as the mitral valve MV) is further reduced. The size of the valve annulus 300 can be contracted or reduced in a wide variety of different ways. Figures 38-43 illustrate the use of a ring or stent 4700 to reduce or contract the valve annulus, but other devices and methods could be employed. Any valved regurgitation reduction device 1034 or combination of the valved regurgitation reduction devices 1034 described herein and/or coapting elements disclosed by Published Patent Cooperation Treaty Application No. WO 2013/173587 can be used in a valve annulus that has been contracted or reduced in size by a ring, stent, or other device or method.

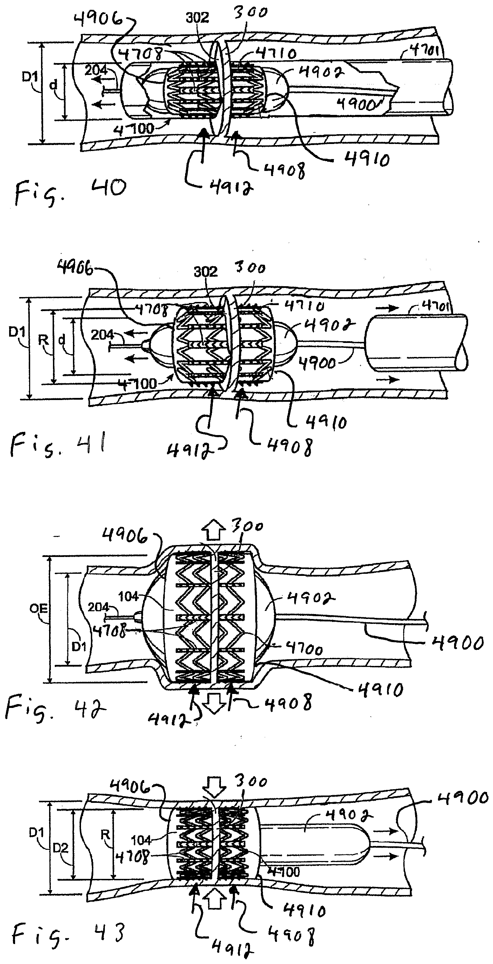

The ring or stent 4700 can take a wide variety of different forms. Figures 40-43 are modified versions of figures from US Patent No. 8,870,949 to Rowe. In one embodiment, a device or devices disclosed by US Patent No. 8,870,949 is used or is modified to be used to contract or reduce the size of a heart valve, such as the tricuspid valve TV or the mitral valve MV. Figures 40-43 illustrate delivery of a ring or stent 4700. In the illustrated embodiment from US Patent No. 8,870,949 to Rowe, the stent or ring 4700 is introduced and positioned across the valve annulus 300 by being inserted at least partially through native valve leaflets 302 and expanded. However, the valved regurgitation reduction devices 1034 disclosed in this application and the coapting devices disclosed by Published Patent Cooperation Treaty Application No. WO 2013/173587, act in conjunction with the native valve leaflets 302, instead of completely replacing the functionality of the native valve leaflets 302. As such, in one embodiment of the present application, the ring or stent 4700 is positioned such that a distal end 4906 is at a position 4908 that is before the annulus 300 (see also Figure 38) or is positioned such that a proximal end 4910 is at a position 4912 that is after the annulus. This leaves the leaflets 302 of the heart valve intact, while still reducing or contracting the valve annulus 300. In another embodiment, two separate rings or stents 4700 are used, with one ring or stent at a position 4908 that is before the annulus 300 and one ring or stent 4700 positioned at a position 4912 that is after the annulus.

Referring to Figures 40-43, in one embodiment, the ring or stent 4700 may be introduced into the patient's body using a percutaneous delivery technique with the balloon portion 4902 of the balloon catheter 4900 deflated, and the ring or stent 4700 operably disposed thereon. The ring or stent 4700 can be contained in a radially crimped or compressed state. In embodiments using a self-expandable stent or ring 4700, the stent or ring 4700 can be held in a compressed state for delivery, by, for example, containing the stent or ring 4700 within an outer covering or sheath 4701. The outer covering 4701 can be removed or retracted, or the stent or ring 4700 pushed through the outer covering 4701, to allow the self-expandable stent or ring 4700 to self-expand. In embodiments having a stent or ring 4700 that does not self-expand, such an outer covering may not be needed to retain the ring or stent 4700 in a crimped state, but can nonetheless be used if desired (e.g. to reduce friction during delivery).

In the embodiment illustrated in Figure 40, the stent or ring 4700 includes projections 4710 of a grabbing mechanisms 4708 are disposed around the outside circumference stent or ring 4700. The stent or ring 4700 is introduced and positioned with respect to the valve annulus 300 and expanded. A diameter D1 of the regurgitant valve 300, 302 is larger than the diameter of a healthy valve in Figures 40-43.

As shown in Figure 41, outer sheath or covering 4701 can be retracted or removed from over the stent or ring 4700. In embodiments having a stent or ring 4700 comprising a shape memory alloy, the stent or ring 4700 can expand from its crimped or compressed diameter d to a predetermined or memorized diameter R once the sheath 201 is removed.

As shown in Figure 42, the balloon portion 4902 of the balloon catheter 4900 is expanded to increase the diameter of the stent or ring 4700 from its relaxed diameter R (Figure 41) to an over-expanded diameter OE such that the outer diameter of the stent or ring 4700 equals or exceeds the original diameter D1 of the annulus 300. In this manner, the annulus 300 may expand beyond the diameter D1 as well. During the expansion, the projections 4710 of the grabbing mechanisms 4708 are forced to contact and can penetrate or otherwise engage (e.g. clamp or grab) the target tissue, which may include tissue on one or both sides of the annulus 300. This causes the stent or ring 4700 to adhere to the tissue on one or both sides of the annulus 300.

Next, as shown in Figure 43, the balloon portion 4902 of the balloon catheter 4900 can be deflated, and the balloon catheter 4900 removed. In embodiments where the stent or ring 4700 is formed of a shape memory material, removing the expansion force of balloon 4902 from the stent or ring 4700 allows the stent or ring 4700 to return from an over-expanded diameter OE (Figure 42) to a recoil or relaxed diameter R or some diameter between the over-expanded diameter OE and the recoil or relaxed diameter R. In one embodiment, the diameter that the ring or stent 4700 returns to is closer to the relaxed diameter R than the over-expanded diameter OE. The manufacture of the ring or stent 4700 determines what the recoil diameter will be. For example, the recoil diameter of a support structure comprising a shape memory alloy can be the memorized or functional diameter of the support structure. The recoil diameter of a support structure comprising, for example, stainless steel and/or cobalt chromium can be that of the natural or resting diameter of the support structure, once it inherently recoils from being over-expanded by the balloon 4902. As the diameter of ring or stent 4700 decreases to the recoil diameter R, the diameter of the annulus 300 also decreases to a final diameter D2. The annulus 300 decreases in diameter due to the projections 4710 of the ring or stent 4700 pulling the target tissue inward.

In one embodiment, the ring or stent 4700 is installed at the same time or a different time than the valved coapting device 1034. For example, the ring or stent 4700 can be installed in the patient three to six months prior to installation of the valved coapting device 1034 or a prosthetic replacement valve (TTVR). This time allows tissue to grow into the ring or stent to form a stable or solid prosthetic annulus. The ring or stent 4700 may be coated to promote tissue growth. For example, the ring or stent 4700 may be coated with a polymer, such as Dacron, etc. to promote tissue growth. In one embodiment, the valved coapting device 1034 or coapting devices disclosed by Published Patent Cooperation Treaty Application No. WO 2013/173587 may be installed in the prosthetic orifice at the same time as the ring 4700. If regurgitation of the valve continues or worsens over time, the valved coapting device 1034 or a coapting device disclosed by Published Patent Cooperation Treaty Application No. WO 2013/173587 can be easily removed and the ring or stent 4700 provides a solid prosthetic seat for a prosthetic valve that replaces the regurgitant valve, instead of working with the regurgitant valve.

## Claims

1. A valved regurgitation reduction system for a native heart valve having native leaflets and an annulus, the valved regurgitation reduction system comprising:
a ring (4700) configured to reduce a size of the annulus;
a valved regurgitation reduction device (1034) that includes:
a coapting element (34) configured to act in conjunction with the native leaflets (302);
a valve (1000) coupled to the coapting element (34);
an anchor (24) configured to position the valved regurgitation reduction device (1034) between the leaflets of a native heart valve;
wherein the coapting element (34) is configured with respect to the native heart valve with the reduced annulus size to form a gap between the leaflets of the native heart valve and the coapting element (34) when the heart is in a diastolic phase and such that the leaflets of the native heart valve seal against the coapting element (34) when the heart is in the systolic phase; and
wherein the valve (1000) coupled to the coapting element (34) is configured to open and allow flow through the coapting element (34) when the heart is in a diastolic phase and to close and prevent flow through the coapting element (34) when the heart is in the systolic phase.

2. The valved regurgitation reduction system of claim 1, wherein the coapting element (34) is configured to form a gap between native tricuspid valve and the coapting element (34) when the heart is in a diastolic phase and such that the leaflets of the native tricuspid valve seal against the coapting element (34) when the heart is in the systolic phase.

3. The valved regurgitation reduction system of claim 1, wherein the coapting element (34) is configured to form a gap between native mitral valve and the coapting element (34) when the heart is in a diastolic phase and such that the leaflets of the native mitral valve seal against the coapting element (34) when the heart is in the systolic phase.

4. The valved regurgitation reduction system of claim 1, wherein the coapting element (34) and the valve (1000) coupled to the coapting element (34) are expandable to allow the valved regurgitation reduction device (1034) to be transvascularly deployed.

5. The valved regurgitation reduction system of claim 1, wherein the valve (1000) coupled to the coapting element (34) is a tri-leaflet type valve.

6. The valved regurgitation reduction system of claim 1, wherein the valve (1000) coupled to the coapting element (34) is disposed in the coapting element (34).

## Patentansprüche

1. System mit Herzklappe zur Regurgitationsreduktion für eine natürliche Herzklappe, die natürliche Klappensegel und einen Anulus aufweist, wobei das System mit Herzklappe zur Regurgitationsreduktion umfasst:
einen Ring (4700), der eingerichtet ist, die Größe des Anulus zu reduzieren;
eine Regurgitationsreduktionsvorrichtung (1034) mit Herzklappe, die enthält:
ein Koaptationselement (34), das eingerichtet ist, zusammen mit den natürlichen Klappensegeln (302) zu wirken;
eine mit dem Koaptationselement (34) gekoppelte Herzklappe (1000);
einen Anker (24), der eingerichtet ist, die Regurgitationsreduktionsvorrichtung (1034) mit Herzklappe zwischen den Klappensegeln einer natürlichen Herzklappe zu positionieren;
wobei das Koaptationselement (34) bezogen auf die natürliche Herzklappe mit der reduzierten Anulusgröße eingerichtet ist, eine Lücke zwischen den Klappensegeln der natürlichen Herzklappe und dem Koaptationselement (34) zu bilden, wenn das Herz in einer diastolischen Phase ist, und so, dass die Klappensegel der natürlichen Herzklappe gegen das Koaptationselement (34) abdichten, wenn das Herz in der systolischen Phase ist; und
wobei die mit dem Koaptationselement (34) gekoppelte Herzklappe (1000) eingerichtet ist, zu öffnen und einen Fluss durch das Koaptationselement (34) zu ermöglichen, wenn das Herz in einer diastolischen Phase ist, und zu Schließen und Fluss durch das Koaptationselement (34) zu verhindern, wenn das Herz in der systolischen Phase ist.

2. System mit Herzklappe zur Regurgitationsreduktion nach Anspruch 1, wobei das Koaptationselement (34) eingerichtet ist, eine Lücke zwischen der natürlichen Trikuspidalklappe und dem Koaptationselement (34) zu bilden, wenn das Herz in einer diastolischen Phase ist, so dass die Klappensegel der natürlichen Trikuspidalklappe gegen das Koaptationselement (34) abdichten, wenn das Herz in der systolischen Phase ist.

3. System mit Herzklappe zur Regurgitationsreduktion nach Anspruch 1, wobei das Koaptationselement (34) eingerichtet ist, eine Lücke zwischen der natürlichen Mitralklappe und dem Koaptationselement (34) zu bilden, wenn das Herz in einer diastolischen Phase ist, so dass die Klappensegel der natürlichen Mitralklappe gegen das Koaptationselement (34) abdichten, wenn das Herz in der systolischen Phase ist.

4. System mit Herzklappe zur Regurgitationsreduktion nach Anspruch 1, wobei das Koaptationselement (34) und die mit dem Koaptationselement (34) gekoppelte Herzklappe (1000) expandierbar sind, damit der Regurgitationsreduktionsvorrichtung mit Herzklappe (1034) ermöglicht wird, transvaskular eingesetzt zu werden.

5. System mit Herzklappe zur Regurgitationsreduktion nach Anspruch 1, wobei die mit dem Koaptationselement (34) gekoppelte Herzklappe (1000) ein Herzklappentyp mit drei Klappensegeln ist.

6. System mit Herzklappe zur Regurgitationsreduktion nach Anspruch 1, wobei die mit dem Koaptationselement (34) gekoppelte Herzklappe (1000) in dem Koaptationselement (34) angeordnet ist.

## Revendications

1. Système de réduction de régurgitation valvulaire destiné à une valvule cardiaque native ayant des feuillets natifs et un anneau, système de réduction de régurgitation valvulaire comprenant :
une bague (4700) configurée pour réduire une taille de l'anneau ;
un dispositif de réduction de régurgitation valvulaire (1034) qui comporte :
un élément de coaptation (34) configuré pour agir en conjonction avec les feuillets natifs (302) ;
une valvule (1000) couplé à l'élément de coaptation (34)
un ancrage (24) configuré pour positionner le dispositif de réduction de regurgitation valvulaire (1034) entre les feuillets d'une valvule cardiaque native ;
dans lequel l'élément de coaptation (34) est configuré par rapport à la valvule cardiaque native avec la taille d'anneau réduite pour former un espace entre les feuillets de la valvule cardiaque native et l'élément de coaptation (34) lorsque le coeur est dans une phase diastolique et de telle sorte que les feuillets de la valvule cardiaque native se scellent contre l'élément de coaptation (34) lorsque le coeur est dans la phase systolique ; et
dans lequel la valvule (1000) couplée à l'élément de coaptation (34) est configurée pour s'ouvrir et permettre un écoulement à travers l'élément de coaptation (34) lorsque le coeur est dans une phase diastolique et pour se fermer et empêcher un écoulement à travers l'élément de coaptation (34) lorsque le coeur est dans la phase systolique.

2. Système de réduction de régurgitation valvulaire selon la revendication 1, dans lequel l'élément de coaptation (34) est configuré pour former un espace entre une valvule tricuspide native et l'élément de coaptation (34) lorsque le coeur est dans une phase diastolique et de telle sorte que les feuillets de la valvule tricuspide native se scellent contre l'élément de coaptation (34) lorsque le coeur est dans la phase systolique.

3. Système de réduction de régurgitation valvulaire selon la revendication 1, dans lequel l'élément de coaptation (34) est configuré pour former un espace entre une valvule mitrale native et l'élément de coaptation (34) lorsque le coeur est dans une phase diastolique et de telle sorte que les feuillets de la valvule mitrale native se scellent contre l'élément de coaptation (34) lorsque le coeur est dans la phase systolique.

4. Système de réduction de régurgitation valvulaire selon la revendication 1, dans lequel l'élément de coaptation (34) et la valvule (1000) couplée à l'élément de coaptation (34) sont extensibles pour permettre au dispositif de réduction de régurgitation valvulaire (1034) d'être déployé par voie transvasculaire.

5. Système de réduction de régurgitation valvulaire selon la revendication 1, dans lequel la valvule (1000) couplée à l'élément de coaptation (34) est une valvule de type à trois feuillets.

6. Système de réduction de régurgitation valvulaire selon la revendication 1, dans lequel la valvule (1000) couplée à l'élément de coaptation (34) est disposé dans l'élément de coaptation (34).
